# EUROPEAN PATENT APPLICATION

(11) **EP 2 298 303 A1**
(43) Date of publication of application: **23.03.2011**
(21) Application number: 10159824.1
(22) Date of filing: 09.09.2004
(51) Int. Cl.: A61K 31/485, A61K 9/00, A61K 9/22, A61P 25/36

(54) **Pharmaceutical combinations of hydrocodone and naltrexone**

(30) Priority: 25.09.2003 US 506222 P
(62) Divisional of application: 04788669.2
(71) Applicant: EURO-CELTIQUE S.A., 1653 Luxembourg (LU)
(72) Inventor: Oshlack, Benjamin, Boca Raton, FL 33432 (US); Wright, Curtis, Rockport, MA 01966 (US); Breder, Chris, Greenwich, CT 06830 (US)
(74) Representative: Bühler, Dirk

(57) **Abstract**

Disclosed is a pharmaceutical composition comprising from about 5 to about 20 mg of hydrocodone or a pharmaceutically acceptable salt thereof and from 0.055 to about 0.56 mg naltrexone or pharmaceutically acceptable salt thereof

## Description

### BACKGROUND OF THE INVENTION

Hydrocodone formulations are sometimes the subject of abuse. A particular dose of hydrocodone may be more potent when administered parenterally as compared to the same dose administered orally. One mode of abuse of oral hydrocodone formulations involves putting the active agent in solution and injecting it.

In the prior art, opioid antagonists have been combined with certain opioid agonists in order to deter the parenteral abuse of these drugs.

The combination of immediate release pentazocine and naloxone has been utilized in tablets available in the United States, commercially available as Talwin^{®}Nx from Sanofi-Winthrop. Talwin^{®}Nx contains immediate release pentazocine hydrochloride equivalent to 50 mg base and naloxone hydrochloride equivalent to 0.5 mg base. A fixed combination therapy comprising tilidine (50 mg) and naloxone (4 mg) has been available in Germany for the management of pain since 1978 (Valoron^{®}N, Goedecke). A fixed combination of buprenorphine and naloxone was introduced in 1991 in New Zealand (Temgesic^{®}Nx, Reckitt & Colman) for the treatment of pain.

U.S. Patent No. 4,769,372 and 4,785,000 to Kreek describe methods of treating patients suffering from chronic pain or chronic cough without provoking intestinal dysmotility by administering 1 to 2 dosage units comprising from about 1.5 to about 100 mg of opioid analgesic or antitussive and from about 1 to about 18 mg of an opioid antagonist having little to no systemic antagonist activity when administered orally, from 1 to 5 times daily.

U.S. Patent No. 5,472,943 to Crain et al. describes methods of enhancing the analgesic potency of bimodally acting opioid agonists by administering the agonist with an opioid antagonist.

Hydrocodone is commercially available in combination with acetaminophen and indicated for the treatment of pain under the tradenames Anexsia® by Mallinckrodt, Lortab® by UCB Pharma, Norco® by Watson Pharmaceuticals, Vicodin® by Abbott, and Zydone® by Endo Labs.

### OBJECTS AND SUMMARY OF THE INVENTION

It is an object of the invention to provide an oral dosage form of hydrocodone.

It is an object of certain embodiments of the invention to provide an oral dosage form of hydrocodone which is subject to less parenteral and/or oral abuse than other dosage forms.

It is an object of certain embodiments of the invention to provide an oral dosage form of hydrocodone which is subject to less diversion than other dosage forms.

It is an object of certain embodiments of the invention to provide a method of treating pain in human patients with an oral dosage form of hydrocodone while reducing the abuse potential of the dosage form.

It is an object of certain embodiments of the invention to provide a method of manufacturing an oral dosage form of hydrocodone such that it has less abuse potential.

These objects and others are achieved by the present invention, which is directed in part to a pharmaceutical composition comprising from 5 to 20 mg of hydrocodone or a pharmaceutically acceptable salt thereof and 0.055 to 0.56 mg naltrexone or a pharmaceutically acceptable salt thereof, the ratio of naltrexone or pharmaceutically acceptable salt thereof to said hydrocodone or pharmaceutically acceptable salt thereof being from 0.011:1 to 0.028:1.

In certain embodiments, the invention is directed to a pharmaceutical composition comprising about 5 mg hydrocodone or a pharmaceutically acceptable salt thereof and 0.055 to 0.14 mg naltrexone or a pharmaceutically acceptable salt thereof.

In certain embodiments, the invention is directed to a pharmaceutical composition comprising about 7.5 mg hydrocodone or a pharmaceutically acceptable salt thereof and 0.0825 to 0.21 mg naltrexone or a pharmaceutically acceptable salt thereof.

In certain embodiments, the invention is directed to a pharmaceutical composition comprising about 10 mg hydrocodone or a pharmaceutically acceptable salt thereof and 0.11 to 0.28 mg naltrexone or a pharmaceutically acceptable salt thereof.

In certain embodiments, the invention is directed to a pharmaceutical composition comprising about 15 mg hydrocodone or a pharmaceutically acceptable salt thereof and 0.165 to 0.42 mg naltrexone or a pharmaceutically acceptable salt thereof.

In certain embodiments, the invention is directed to a pharmaceutical composition comprising about 20 mg hydrocodone or a pharmaceutically acceptable salt thereof and 0.22 to 0.56 mg naltrexone or a pharmaceutically acceptable salt thereof.

In certain embodiments of the invention disclosed herein, the dosage form provides sustained release of the hydrocodone, the naltrexone, or a sustained release of both agents.

In certain embodiments of the invention disclosed herein, the dosage form provides effective pain relief for at least 12 hours after steady state oral administration to human patients.

In certain embodiments of the invention disclosed herein, the dosage form provides effective pain relief for at least 24 hours after steady state oral administration to human patients.

In certain embodiments of the invention disclosed herein, the dosage form comprises a matrix comprising the hydrocodone or pharmaceutically acceptable salt thereof and the naltrexone or pharmaceutically acceptable salt thereof, wherein both the hydrocodone or pharmaceutically acceptable salt thereof and naltrexone or pharmaceutically acceptable salt thereof are substantially interdispersed in a sustained release excipient.

In certain embodiments, the invention is directed to a method of reducing the potential of parenteral abuse of a hydrocodone formulation comprising preparing the compositions disclosed herein.

In certain embodiments, the invention is directed to a method of treating pain in a human patient comprising orally administering a pharmaceutical composition as disclosed herein that provides effective pain relief for at least 12 hours after steady state oral administration to the patient.

In certain embodiments, the invention is directed to a method of treating pain in a human patient comprising orally administering a pharmaceutical composition as disclosed herein that provides effective pain relief for at least 24 hours after steady state oral administration to the patient.

The term "sustained release" is defined for purposes of the present invention as the release of the hydrocodone or salt thereof from the dosage form at such a rate that blood (e.g., plasma) concentrations (levels) are maintained within the therapeutic range (above the minimum effective analgesic concentration or "MEAC") but below toxic levels over a period of 8 to 24 hours, preferably over a period of time indicative of a twice-a-day or a once-a-day formulation.

The term "parenterally" as used herein includes subcutaneous injections, intravenous, intramuscular, intrasternal injection, infusion techniques or other methods of injection known in the art.

Unless otherwise noted, the term "hydrocodone" means hydrocodone base. Unless otherwise noted, the term "naltrexone" means naltrexone base. The term salt means a pharmaceutically acceptable salt.

The term "steady state" means that the amount of the drug reaching the system is approximately the same as the amount of the drug leaving the system. Thus at "steady state", the patient's body eliminates the drug at approximately the same rate that the drug becomes available to the patient's system through absorption into the blood stream.

### BRIEF DESCRIPION OF THE DRAWING

Figure 1 depicts the maximum change from baseline (PDmax) for the subjective drug effect "Liking This Feeling" for each of the three treatment periods of Example 6.

Figure 2 depicts the area under curve (AUC) for the PDmax for the subjective drug effect "Liking This Feeling" for each of the three treatment periods of Example 6.

Figure 3 depicts the maximum change from baseline (PDmax) for the subjective drug effect "Good Effects" for each of the three treatment periods of Example 6.

Figure 4 depicts the area under curve (AUC) for the PDmax for the subjective drug effect "Good Effects" for each of the three treatment periods of Example 6.

Figure 5 depicts the maximum change from baseline (PDmax) for the subjective drug effect "Feeling Sick" for each of the three treatment periods of Example 6.

Figure 6 depicts the area under curve (AUC) for the PDmax for the subjective drug effect "Feeling Sick" for each of the three treatment periods of Example 6.

Figure 7 depicts the maximum change from baseline (PDmax) for the subjective drug effect "Bad Effects" for each of the three treatment periods of Example 6.

Figure 8 depicts the area under curve (AUC) for the PDmax for the subjective drug effect "Bad Effects" for each of the three treatment periods of Example 6.

Figure 9 depicts the maximum change from baseline (PDmax) for the subjective "Antagonist Total Sympton Score" for each of the three treatment periods of Example 6.

Figure 10 depicts the area under curve (AUC) for the PDmax for the subjective "Antagonist Total Sympton Score" for each of the three treatment periods of Example 6.

Figure 11 depicts the maximum change from baseline (PDmax) for pupil diameter for each of the three treatment periods of Example 6.

Figure 12 depicts the area under curve (AUC) for the PDmax for pupil diameter for each of the three treatment periods of Example 6.

### DETAILED DESCRIPTION OF THE INVENTION

The dosage form of the present invention contains from about 5 to about 20 mg of hydrocodone or a pharmaceutically acceptable salts thereof. Particularly preferred dosages of hydrocodone or salt thereof are about 5 mg, about 7.5 mg, about 10 mg, about 15 mg and about 20 mg. In certain embodiments, the hydrocodone or salt thereof is formulated with suitable pharmaceutically acceptable excipients to provide a sustained release of the hydrocodone.

The dosage form of the present invention contains about 0.055 to about 0.56 mg of naltrexone or pharmaceutically acceptable salts thereof. Particularly preferred dosages of naltrexone or salt thereof are about 0.0625 mg, about .09375 mg, about 0.125 mg, about 0.1875 mg and about 0.25 mg.

The hydrocodone or salt thereof and naltrexone or salt thereof can be formulated to provide immediate release of one or both agents or can be combined with suitable pharmaceutically acceptable excipients to provide a sustained release of one or both agents. The rate of sustained release of the naltrexone or salt thereof can be the same or different than the rate of sustained release of the hydrocodone or salt thereof. Particularly preferred embodiments of the present invention are dosage forms which comprise about 5 mg hydrocodone salt and about 0.0625 mg naltrexone salt; about 7.5 mg hydrocodone salt and about 0.09375 mg naltrexone salt; about 10 mg hydrocodone salt and about 0.125 mg naltrexone salt; about 15 mg hydrocodone salt and about 0.1875 mg naltrexone salt; and about 20 mg hydrocodone salt and about 0.25 mg naltrexone salt. Bitartrate salts of hydrocodone and hydrochloride salts of naltrexone are particularly preferred.

In certain embodiments of the invention, the disclosed range of naltrexone or salt thereof may be in an amount sufficient to deter intranasal and parenteral abuse of the formulation in physically dependent subjects by at least partially blocking the opioid effects of the hydrocodone if the formulation is tampered with and administered to the nasal mucosa or administered parenterally. Preferably the amount is also sufficient so that intranasal or parenteral administration in most physically dependent individuals results in precipitation of a moderate to severe withdrawal syndrome that is very similar to that seen after abrupt withdrawal of opioids. The most common symptoms of the withdrawal syndrome include pupillary dilation, chills alternating with excessive sweating, abdominal cramps, nausea, vomiting, muscle spasms, hyperirritability, lacrimation, rinorrhea, goose flesh and increased heart rate.

In certain embodiments a stabilizer is included in the dosage form to prevent the degradation of the naltrexone or pharmaceutically acceptable salt thereof. In certain embodiments, stabilizers of use in the dosage form include for example and without limitation, organic acids, carboxylic acids, acid salts of amino acids (e.g., cysteine, L-cysteine, cysteine hydrochloride, glycine hydrochloride or cystine dihydrochloride), sodium metabisulphite, ascorbic acid and its derivatives, malic acid, isoascorbic acid, citric acid, tartaric acid, palmitic acid, sodium carbonate, sodium hydrogen carbonate, calcium carbonate, calcium hydrogen phosphate, sulphur dioxide, sodium sulphite, sodium bisulphate, tocopherol, as well as its water- and fat-soluble derivatives, such as e.g., tocofersolan or tocopherol acetate, sulphites, bisulphites and hydrogen sulphites or alkali metal, alkaline earth metal and other metals, PHB esters, gallates, butylated hydroxyanisol (BHA) or butylated hydroxytoluene (BHT), and 2,6-di-t-butyl-.alpha.-dimethylamino-p-cresol, t-butylhydroquinone, di-t-amylhydroquinone, di-t-butylhydroquinone, butylhydroxytoluene, butylhydroxyanisole, pyrocatechol, pyrogallol, propyl/gallate, and nordihydroguaiaretic acid, as well as lower fatty acids, fruit acids, phosphoric acids, sorbic and benzoic acids as well as their salts, esters, derivatives and isomeric compounds, ascorbyl palmitate, lecithins, mono- and polyhydroxylated benzene derivatives, ethylenediamine-tetraacetic acid and its salts, citraconic acid, conidendrine, diethyl carbonate, methylenedioxyphenols, kephalines, (β,β'-dithiopropionic acid, biphenyl and other phenyl derivatives, pharmaceutically acceptable salts thereof, and mixtures thereof.

The oral dosage form of the present invention may further include, in addition to the hydrocodone and naltrexone, one or more drugs that may or may not act synergistically therewith. Thus, in certain embodiments, a non-opioid drug is also included in the formulation. Such non-opioid drugs would preferably provide additional analgesia, and include, for example, aspirin, non-steroidal anti-inflammatory drugs ("NSAIDS"), e.g., ibuprofen, ketoprofen, etc., N-methyl-D-aspartate (NMDA) receptor antagonists, e.g., a morphinan such as dextromethorphan or dextrorphan, or ketamine, cycooxygenase-II inhibitors ("COX-II inhibitors"), and/or glycine receptor antagonists, among others.

In certain preferred embodiments of the present invention, the invention allows for the use of lower doses of the hydrocodone by virtue of the inclusion of an additional non-opioid analgesic, such as an NSAID or a COX-2 inhibitor. By using lower amounts of either or both drugs, the side effects associated with effective pain management in humans can be reduced.

Suitable non-steroidal anti-inflammatory agents, include ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam or isoxicam, pharmaceutically acceptable salts thereof, mixtures thereof, and the like. Useful dosages of these drugs are well known to those skilled in the art.

N-methyl-D-aspartate (NMDA) receptor antagonists are well known in the art, and encompass, for example, morphinans such as dextromethorphan or dextrorphan, ketamine, d-methadone and pharmaceutically acceptable salts thereof. For purposes of the present invention, the term "NMDA antagonist" is also deemed to encompass drugs that block an intracellular response of NMDA-receptor activation, e.g. a ganglioside such as GM, or GT_{1b}a phenothiazine such as trifluoperazine or a naphthalene-sulfonamide such as N-(6-aminothexyl)-5-chloro-1-naphthalenesulfonamide. These drugs are stated to inhibit the development of tolerance to and/or dependence on addictive drugs, e.g., narcotic analgesics such as morphine, codeine, etc. in U.S. Pat. Nos. 5,321,012 and 5,556,838 (both to Mayer, et al.), and to treat chronic pain in U.S. Pat. No. 5,502,058 (Mayer, et al.), all of which are hereby incorporated by reference. The NMDA antagonist may be included alone, or in combination with a local anesthetic such as lidocaine, as described in the patents to Mayer et al.

The treatment of chronic pain via the use of glycine receptor antagonists and the identification of such drugs is described in U.S. Pat. No. 5,514,680 (Weber, et al.).

COX-2 inhibitors have been reported in the art and many chemical structures are known to produce inhibition of cyclooxygenase-2. COX-2 inhibitors are described, for example, in U.S. Patent Nos. 5,616,601; 5,604,260; 5,593,994; 5,550,142; 5,536,752; 5,521,213; 5,475,995; 5,639,780; 5,604,253; 5,552,422; 5,510,368; 5,436,265; 5,409,944; and 5,130,311, all of which are hereby incorporated by reference. Certain preferred COX-2 inhibitors include celecoxib, 5-bromo-s-(4-fluorophenyl)-3-[4-(methylsufonyl)phenyl] thiophene, flosulide , meloxicam, rofecoxib , 6-methoxy-2 naphthylacetic acid , nabumetone, nimesulide, N-[2-(cyclohexyloxy)-4-nitrophenyl] methanesulfonamide, 1 -fluoro-4-[2-[4-(methylsufonyl)phenyl]-l-cyclopenten-1-yl] benzene, 5-(4-fluorophenyl)-1-[4-(methylsufonyl)phenyl]-3-trifluoromethyl 1H-pyrazole, N-[3-(formylamino)-4-oxo-6-phenoxy-4H-1-benzopyran-7-ylJ methanesulfonamide, mixtures thereof, and pharmaceutically acceptable salts thereof. Dosage levels of a COX-2 inhibitor on the order of from about 0.005 mg to about 140 mg per kilogram of body weight per day are therapeutically effective in combination with an opioid analgesic. Alternatively, about 0.25 mg to about 7 g per patient per day of a COX-2 inhibitor is administered in combination with an opioid analgesic.

In yet further embodiments, a non-opioid drug can be included which provides a desired effect other than analgesia, e.g., antitussive, expectorant, decongestant, antihistamine drugs, local anesthetics, and the like.

### SUSTAINED RELEASE DOSAGE FORMS

The hydrocodone (or hydrocodone salt) and/or the naltrexone (or naltrexone salt) may be formulated as a sustained release oral formulation in any suitable tablet, coated tablet or multiparticulate formulation known to those skilled in the art. The sustained release dosage form may include a sustained release material which is incorporated into a matrix along with the hydrocodone or salt thereof with or without the naltrexone or salt thereof. For example, hydrocodone salt can be incorporated in a sustained release matrix and naltrexone salt can be separate from the matrix or can be incorporated into the matrix.

The sustained release dosage form in certain embodiments may comprise one group of particles containing both the hydrocodone or salt thereof and the naltrexone or salt thereof. In other embodiments, the dosage form may comprise one group of particles containing the hydrocodone or salt thereof and a second group of particles containing the naltrexone or salt thereof. In embodiments with one or multiple groups of particles, the particles can have a diameter from about 0.1 mm to about 2.5 mm, preferably from about 0.5 mm to about 2 mm. As disclosed above, the naltrexone or naltrexone salt may be incorporated into particles which contain hydrocodone or hydrocodone salt, may be incorporated into separate particles, or may be incorporated into a tablet or capsule containing hydrocodone or hydrocodone salt particles. In certain embodiments, the particles are coated with a sustained release material that permits release of the active(s) at a sustained rate in an aqueous medium. The coat is chosen so as to achieve, in combination with the other stated properties, a desired in-vitro release rate. The sustained release coating formulations of the present invention should be capable of producing a strong, continuous film that is smooth and elegant, capable of supporting pigments and other coating additives, non-toxic, inert, and tack―free.

### COATED BEADS

In certain embodiments of the present invention a hydrophobic material is used to overcoat active agent coated inert pharmaceutical beads, such as nu pariel 18/20 beads. A plurality of the resultant solid sustained release beads may thereafter be placed in a gelatin capsule in an amount sufficient to provide an effective sustained release dose when ingested and contacted by an environmental fluid, e.g., gastric fluid or dissolution media. In certain embodiments, a sustained release bead containing hydrocodone or hydrocodone salt may be further coated with naltrexone or a naltrexone salt. Alternatively, the naltrexone or naltrexone salt may be placed in a capsule with the sustained release hydrocodone or hydrocodone salt beads (e.g., as a powder mixture or formulated into separate beads).

The sustained release bead formulations of the present invention slowly release the active agent(s) of the present invention, e.g., when ingested and exposed to gastric fluids, and then to intestinal fluids. The sustained release profile of the formulations of the invention can be altered, for example, by varying the amount of overcoating with the hydrophobic material, altering the manner in which a plasticizer is added to the hydrophobic material, by varying the amount of plasticizer relative to hydrophobic material, by the inclusion of additional ingredients or excipients, by altering the method of manufacture, etc. The dissolution profile of the ultimate product may also be modified, for example, by increasing or decreasing the thickness of the retardant coating.

Spheroids or beads coated with the active agent(s) of the present are prepared, e.g., by dissolving the active agent(s) in water and then spraying the solution onto a substrate, for example, nu pariel 18/20 beads, using a Wuster insert. Optionally, additional ingredients are also added prior to coating the beads in order to assist the binding of the agent(s) to the beads, and/or to color the solution, etc. For example, a product which includes hydroxypropylmethylcellulose, etc., with or without colorant (e.g., Opadry^{®}, commercially available from Colorcon, Inc.) may be added to the solution and the solution mixed (e.g., for about 1 hour) prior to application of the same onto the beads. The resultant coated substrate, in this example beads, may then be optionally overcoated with a barrier agent to separate the active agent(s) , from the hydrophobic sustained release coating. An example of a suitable barrier agent is one which comprises hydroxypropylmethylcellulose. However, any film-former known in the art may be used. It is preferred that the barrier agent does not affect the dissolution rate of the final product.

The beads may then be overcoated with an aqueous dispersion of the hydrophobic material. The aqueous dispersion of hydrophobic material preferably further includes an effective amount of plasticizer, e.g. triethyl citrate. Pre-formulated aqueous dispersions of ethylcellulose, such as Aquacoat^{®} or Surelease^{®}, may be used. If Surelease^{®} is used, it is not necessary to separately add a plasticizer. Alternatively, pre-formulated aqueous dispersions of acrylic polymers such as Eudragit^{®} can be used.

The coating solutions of the present invention preferably contain, in addition to the film-former, plasticizer, and solvent system (i.e., water), a colorant to provide elegance and product distinction. Color may be added to the solution of the active agent instead of, or in addition to the aqueous dispersion of hydrophobic material. For example, color may be added to Aquacoat^{®} via the use of alcohol or propylene glycol based color dispersions, milled aluminum lakes and opacifiers such as titanium dioxide by adding color with shear to water soluble polymer solution and then using low shear to the plasticized Aquacoat^{®}. Alternatively, any suitable method of providing color to the formulations of the present invention may be used. Suitable ingredients for providing color to the formulation when an aqueous dispersion of an acrylic polymer is used include titanium dioxide and color pigments, such as iron oxide pigments. The incorporation of pigments, may, however, increase the retard effect of the coating.

Plasticized hydrophobic material may be applied onto the substrate comprising the agent(s) by spraying using any suitable spray equipment known in the art. In a preferred method, a Wurster fluidized-bed system is used in which an air jet, injected from underneath, fluidizes the core material and effects drying while the acrylic polymer coating is sprayed on. A sufficient amount of the hydrophobic material to obtain a predetermined sustained release of the agent(s) when the coated substrate is exposed to aqueous solutions, e.g. gastric fluid, may be applied. After coating with the hydrophobic material, a further overcoat of a film-former, such as Opadry^{®}, is optionally applied to the beads. This overcoat is provided, if at all, in order to substantially reduce agglomeration of the beads.

The release of the agent(s) from the sustained release formulation of the present invention can be further influenced, i.e., adjusted to a desired rate, by the addition of one or more release-modifying agents, or by providing one or more passageways through the coating. The ratio of hydrophobic material to water soluble material is determined by, among other factors; the release rate required and the solubility characteristics of the materials selected.

The release-modifying agents that function as pore-formers may be organic or inorganic, and include materials that can be dissolved, extracted or leached from the coating in an environment of use. The pore-formers may comprise one or more hydrophilic materials such as hydroxypropylmethylcellulose.

The release-modifying agent may also or alternatively comprise a semi-permeable polymer.

In certain preferred embodiments, the release-modifying agent is selected from hydroxypropylmethylcellulose, lactose, metal stearates, and mixtures of any of the foregoing.

The sustained release costings of the present invention can also include erosion-promoting agents such as starch and gums.

The sustained release coatings of the present invention can also include materials useful for making microporous lamina in the environment of use, such as polycarbonates comprised of linear polyesters of carbonic acid in which carbonate groups reoccur in the polymer chain.

The sustained release coatings of the present invention may also include an exit means comprising at least one passageway, orifice, or the like. The passageway may be formed by such methods as those disclosed in U.S. Patent Nos. 3,845,770; 3,916,889; 4,063,064; and 4,088,864. The passageway can have any shape such as round, triangular, square, elliptical, irregular, etc.

### MATRIX FORMULATIONS

In other embodiments of the present invention, the sustained release formulation is achieved via a matrix optionally having a sustained release coating as set forth herein. The materials suitable for inclusion in a sustained release matrix may depend on the method used to form the.matrix.

For example, a matrix in addition to the hydrocodone (or hydrocodone salt) and optional naltrexone (or naltrexone salt) may be selected from: (i) hydrophilic and/or hydrophobic materials, such as gums, cellulose ethers, acrylic polymers or resins, protein derived materials and any pharmaceutically acceptable hydrophobic material or hydrophilic material which is capable of imparting sustained release of the active agent(s) and which melts (or softens to the extent necessary to be extruded) (ii) digestible, long chain (C₈-C₅₀, especially C₁₂-C₄₀), substituted or unsubstituted hydrocarbons, such as fatty acids, fatty alcohols, glyceryl esters of fatty acids, mineral and vegetable oils and waxes, and stearyl alcohol, and (iii) polyalkylene glycols.

Of these polymers, acrylic polymers, especially Eudragit^{®} RSPO, - and the cellulose ethers, especially hydroxyalkylcelluloses and carboxyalkylcelluloses, are preferred. The oral dosage form may contain between 1% and 80% (by weight) of at least one hydrophilic or hydrophobic material.

When the hydrophobic material is a hydrocarbon, the hydrocarbon preferably has a melting point of between 25° and 90°C. Of the long chain hydrocarbon materials, fatty (aliphatic) alcohols are preferred. The oral dosage form may contain up to 60% (by weight) of at least one digestible, long chain hydrocarbon.

Preferably, the oral dosage form contains up to 60% (by weight) of at least one polyalkylene glycol.

The hydrophobic material may be selected from the group consisting of alkylcelluloses, acrylic and methacrylic acid polymers and copolymers, shellac, zein, hydrogenated castor oil, hydrogenated vegetable oil, or mixtures thereof. In certain preferred embodiments of the present invention, the hydrophobic material is a pharmaceutically acceptable acrylic polymer selected from materials such as acrylic acid and methacrylic acid copolymers, methyl methacrylate, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamine copolymer, poly(methyl methacrylate), poly(methacrylic acid)(anhydride), polymethacrylate, polyacrylamide, poly(methacrylic acid anhydride), and glycidyl methacrylate copolymers. In other embodiments, the hydrophobic material is selected from materials such as hydroxyalkylcelluloses such as hydroxypropylmethylcellulose and mixtures of the foregoing.

Preferred hydrophobic materials are water-insoluble with more or less pronounced hydrophilic and/or hydrophobic trends. Preferably, the hydrophobic materials useful in the invention have a melting point from about 30° to about 200°C, preferably from about 45°C to about 90°C. Specifically, the hydrophobic material may comprise natural or synthetic waxes, fatty alcohols (such as lauryl, myristyl, stearyl, cetyl or preferably cetostearyl alcohol), fatty acids, including but not limited to fatty acid esters, fatty acid glycerides (mono-, di-, and tri-glycerides), hydrogenated fats, hydrocarbons, normal waxes, stearic aid, stearyl alcohol and hydrophobic and hydrophilic materials having hydrocarbon backbones. Suitable waxes include, for example, beeswax, glycowax, castor wax and carnauba wax. For purposes of the present invention, a wax-like substance is defined as any material which is normally solid at room temperature and has a melting point of from about 30° to about 100°C.

Suitable hydrophobic materials that may be used in accordance with the present invention include digestible, long chain (C₈-C₅₀, especially C₁₂-C₄₀), substituted or unsubstituted hydrocarbons, such as fatty acids, fatty alcohols, glyceryl esters of fatty acids, mineral and vegetable oils and natural and synthetic waxes. Hydrocarbons having a melting point of between 25° and 90°C are preferred. Of the long chain hydrocarbon materials, fatty (aliphatic) alcohols are preferred in certain embodiments. The oral dosage form may contain up to 60% (by weight) of at least one digestible, long chain hydrocarbon.

Preferably, a combination of two or more hydrophobic materials are included in the matrix formulations. If an additional hydrophobic material is included, it is preferably selected from natural and synthetic waxes, fatty acids, fatty alcohols, and mixtures of the same. Examples include beeswax, carnauba wax, stearic acid and stearyl alcohol. This list is not meant to be exclusive.

One particular suitable matrix comprises at least one water soluble hydroxyalkyl cellulose, at least one C₁₂-C₃₆, preferably C₁₄-C₂₂, aliphatic alcohol and, optionally, at least one polyalkylene glycol. The hydroxyalkyl cellulose is preferably a hydroxy (C₁ - C₆) alkyl cellulose, such as hydroxypropylcellulose, hydroxypropylmethylcellulose or hydroxyethylcellulose. The amount of the hydroxyalkyl cellulose in the present oral dosage form will be determined, inter alia, by the precise rate of hydrocodone and/or naltrexone release required. The aliphatic alcohol may be, for example, lauryl alcohol, myristyl alcohol or stearyl alcohol. In particularly preferred embodiments of the present oral dosage form, however, the aliphatic alcohol is cetyl alcohol or cetostearyl alcohol. The amount of the aliphatic alcohol in the present oral dosage form will be determined, as above, by the precise rate of hydrocodone and/or naltrexone release required. It will also depend on whether at least one polyalkylene glycol is present in or absent from the oral dosage form. In the absence of at least one polyalkylene glycol, the oral dosage form preferably contains between 20% and 50% (by wt) of the aliphatic alcohol. When polyalkylene glycol is present in the oral dosage form, then the combined weight of the aliphatic alcohol and the polyalkylene glycol preferably constitutes between 20% and 50% (by wt) of the total dosage form.

In one embodiment, the ratio of, e.g., the hydroxyalkyl cellulose or acrylic resin to the aliphatic alcohol/polyalkylene glycol determines, to a considerable extent, the release rate of the hydrocodone and/or naltrexone from the formulation. A ratio of the hydroxyalkyl cellulose to the aliphatic alcohol/polyalkylene glycol of between 1:2 and 1:4 is preferred, with a ratio of between 1:3 and 1:4 being particularly preferred.

The polyalkylene glycol may be, for example, polypropylene glycol or polyethylene glycol. The number average molecular weight of the at least one polyalkylene glycol is preferably between 1,000 and 15,000 especially between 1,500 and 12,000.

Another suitable sustained release matrix would comprise an alkylcellulose (especially ethyl cellulose), a C₁₂ to C₃₆ aliphatic alcohol and, optionally, a polyalkylene glycol.

In another preferred embodiment, the matrix includes a pharmaceutically acceptable combination of at least two hydrophobic materials.

In addition to the above ingredients, a sustained release matrix may also contain suitable quantities of other materials, e.g. diluents, lubricants, binders, granulating aids, colorants, flavorants and/or glidants that are conventional in the pharmaceutical art.

### MATRIX - PARTICULATES

In order to facilitate the preparation of a solid, sustained release, oral dosage form according to this invention, any method of preparing a matrix formulation known to those skilled in the art may be used. For example incorporation in the matrix may be effected, for example, by (a) forming granules comprising at least one water soluble hydroxyalkyl cellulose, and the hydrocodone (or hydrocodone salt) and optionally the naltrexone (or naltrexone salt); (b) mixing the resultant granules with at least one C₁₂ - C₃₆ aliphatic alcohol; and (c) optionally, compressing and shaping the granules. Preferably, the granules are formed by wet granulating the hydroxalkyl cellulose granules with water.

In yet other alternative embodiments, a spheronizing agent, together with the hydrocodone (or hydrocodone salt) and optionally the naltrexone (or naltrexone salt) can be spheronized to form spheroids. Microcrystalline cellulose is a preferred spheronizing agent. A suitable microcrystalline cellulose is, for example, the material sold as Avicel PH 101 (Trade Mark, FMC Corporation). In such embodiments, in addition to the active ingredient and spheronizing agent, the spheroids may also contain a binder. Suitable binders, such as low viscosity, water soluble polymers, will be well known to those skilled in the pharmaceutical art. However, water soluble hydroxy lower alkyl cellulose, such as hydroxypropylcellulose, are preferred. Additionally (or alternatively) the spheroids may contain a water insoluble polymer, especially an acrylic polymer, an acrylic copolymer, such as a methacrylic acid-ethyl acrylate copolymer, or ethyl cellulose. In such embodiments, the sustained release coating will generally include a hydrophobic material such as (a) a wax, either alone or in admixture with a fatty alcohol; or (b) shellac or zein.

### Melt Extrusion Matrix

Sustained release matrices can also be prepared via melt-granulation or melt-extrusion techniques. Generally, melt-granulation techniques involve melting a normally solid hydrophobic material, e.g. a wax, and incorporating a powdered drug therein. To obtain a sustained release dosage form, it may be necessary to incorporate an additional hydrophobic substance, e.g. ethylcellulose or a water-insoluble acrylic polymer, into the molten wax hydrophobic material. Examples of sustained release formulations prepared via melt-granulation techniques are found in U.S. Patent No. 4,861,598.

The additional hydrophobic material may comprise one or more water-insoluble wax-like thermoplastic substances possibly mixed with one or more wax-like thermoplastic substances being less hydrophobic than said one or more water-insoluble wax-like substances. In order to achieve constant release, the individual wax-like substances in the formulation should be substantially non-degradable and insoluble in gastrointestinal fluids during the initial release phases. Useful water-insoluble wax-like substances may be those with a water-solubility that is lower than about 1:5,000 (w/w).

In addition to the above ingredients, a sustained release matrix may also contain suitable quantities of other materials, e.g., diluents, lubricants, binders, granulating aids, colorants, flavorants and glidants that are conventional in the pharmaceutical art. The quantities of these additional materials will be sufficient to provide the desired effect to the desired formulation.

In addition to the above ingredients, a sustained release matrix incorporating melt-extruded multiparticulates may also contain suitable quantities of other materials, e.g. diluents, lubricants, binders, granulating aids, colorants, flavorants and/or glidants that are conventional in the pharmaceutical art.

Specific examples of pharmaceutically acceptable carriers and excipients that may be used to formulate oral dosage forms are described in the Handbook of Pharmaceutical Excipients,American Pharmaceutical Association, 3rd ed. (2000).

### Melt Extrusion Multiparticulates

The preparation of a suitable melt-extruded matrix according to the present invention may, for example, include the steps of blending the hydrocodone (or hydrocodone salt) and/or the naltrexone (or naltrexone salt) together with at least one hydrophobic material to obtain a homogeneous mixture. The homogeneous mixture is then heated to a temperature sufficient to at least soften the mixture sufficiently to extrude the same. The resulting homogeneous mixture is then extruded to form strands. The extrudate is preferably cooled and cut into multiparticulates by any means known in the art. The strands are cooled and cut into multiparticulates. The multiparticulates are then divided into unit doses. The extrudate preferably has a diameter of from about 0.1 to about 5 mm and provides sustained release of the active agent for a time period of from about 8 to about 24 hours.

An optional process for preparing the melt extrusions of the present invention includes directly metering into an extruder a hydrophobic material, the hydrocodone (or hydrocodone salt) and optionally the naltrexone (or naltrexone salt), and an optional binder; blending and heating the ingredients to form a homogenous mixture; extruding the homogenous mixture to thereby form strands; cooling the strands containing the homogeneous mixture; cutting the strands into particles having a size from about 0.1 mm to about 12 mm; and dividing said particles into unit doses. In this aspect of the invention, a relatively continuous manufacturing procedure is realized.

The diameter of the extruder aperture or exit port can also be adjusted to vary the thickness of the extruded strands. Furthermore, the exit port of the extruder need not be round; it can be oblong, rectangular, etc. The exiting strands can be reduced to particles using a hot wire cutter, guillotine, etc.

The melt extruded multiparticulate system can be, for example, in the form of granules, spheroids or pellets depending upon the extruder exit port. For purposes of the present invention, the terms "melt-extruded multiparticulate(s)" (MEMS) and "melt-extruded multiparticulate system(s)" and "melt-extruded particles" refer to a plurality of units, preferably within a range of similar size and/or shape and containing one or more active agents and one or more excipients, preferably including a hydrophobic material as described herein. In this regard, the melt-extruded multiparticulates will be of a range of from about 0.1 to about 12 mm in length and have a diameter of from about 0.1 to about 5 mm. In addition, it is to be understood that the melt-extruded multiparticulates can be any geometrical shape within this size range. Alternatively, the extrudate may simply be cut into desired lengths and divided into unit doses of the therapeutically active agent without the need of a spheronization step.

In one preferred embodiment, oral dosage forms are prepared to include an effective amount of melt-extruded multiparticulates within a capsule. For example, a plurality of the melt-extruded multiparticulates may be placed in a gelatin capsule in an amount sufficient to provide an effective sustained release dose when ingested and contacted by gastric fluid.

In another preferred embodiment, a suitable amount of the multiparticulate extrudate is compressed into an oral tablet using conventional tableting equipment using standard techniques. Techniques and compositions for making tablets (compressed and molded), capsules (hard and soft gelatin) and pills are also described in Remington's Pharmaceutical Sciences, (Arthur Osol, editor), 1553-1593 (1980).

In yet another preferred embodiment, the extrudate can be shaped into tablets as set forth in U.S. Patent No. 4,957,681 (Klimesch, et. al.), described in additional detail above.

Optionally, the sustained release melt-extruded multiparticulate systems or tablets can be coated, or the gelatin capsule can be further coated, with a sustained release coating such as the sustained release coatings described above. Such coatings preferably include a sufficient amount of hydrophobic material to obtain a weight gain level from about 2 to about 30 percent, although the overcoat may be greater depending upon the desired release rate, among other things.

The melt-extruded unit dosage forms of the present invention may further comprise combinations of melt-extruded particles (e.g., one group of particles with hydrocodone (or hydrocodone salt) and one group of particles with naltrexone (or naltrexone salt)) before being encapsulated. The unit dosage forms can also further comprise an amount of an immediate release active agent for prompt release. The immediate release agent may be incorporated, e.g., as separate pellets within a gelatin capsule, or may be coated on the surface of the multiparticulates after preparation of the dosage forms (e.g., sustained release coating or matrix-based). The unit dosage forms of the present invention may also contain a combination of sustained release beads and matrix multiparticulates to achieve a desired effect.

The sustained release formulations of the present invention preferably slowly release the agent(s), e.g., when ingested and exposed to gastric fluids, and then to intestinal fluids. The sustained release profile of the melt-extruded formulations of the invention can be altered, for example, by varying the amount of retardant, i.e., hydrophobic material, by varying the amount of plasticizer relative to hydrophobic material, by the inclusion of additional ingredients or excipients, by altering the method of manufacture, etc.

In other embodiments of the invention, the melt extruded material is prepared without the inclusion of the hydrocodone (or hydrocodone salt) and the naltrexone (or naltrexone salt), which can be added thereafter to the extrudate. Such formulations typically will have the agents blended together with the extruded matrix material, and then the mixture would be tableted in order to provide a slow release formulation.

### COATINGS

The dosage forms of the present invention may optionally be coated with one or more materials suitable for the regulation of release or for the protection of the formulation. In one embodiment, coatings are provided to permit either pH-dependent or pH-independent release. A pH-dependent coating serves to release the hydrocodone and/or naltrexone in desired areas of the gastro-intestinal (GI) tract, e.g., the stomach or small intestine, such that an absorption profile is provided which is capable of providing at least about eight hours and preferably about twelve hours to up to about twenty-four hours of analgesia to a patient. When a pH-independent coating is desired, the coating is designed to achieve optimal release regardless of pH-changes in the environmental fluid, e.g., the GI tract. It is also possible to formulate compositions that release a portion of the dose in one desired area of the GI tract, e.g., the stomach, and release the remainder of the dose in another area of the GI tract, e.g., the small intestine.

Formulations according to the invention that utilize pH-dependent coatings may also impart a repeat-action effect whereby unprotected drug is coated over the enteric coat and is released in the stomach, while the remainder, being protected by the enteric coating, is released further down the gastrointestinal tract. Coatings that are pH-dependent include shellac, cellulose acetate phthalate (CAP), polyvinyl acetate phthalate (PVAP), hydroxypropylmethylcellulose phthalate, and methacrylic acid ester copolymers, zein, and the like.

In certain preferred embodiments, the substrate (e.g., coated bead, matrix particle) containing the hydrocodone or salt thereof and optionally the naltrexone or salt thereof is coated with a hydrophobic material selected from (i) an alkylcellulose; (ii) an acrylic polymer; or (iii) mixtures thereof. The coating may be applied in the form of an organic or aqueous solution or dispersion. The coating may be applied to obtain a weight gain from about 2 to about 25% of the substrate in order to obtain a desired sustained release profile. Coatings derived from aqueous dispersions are described, e.g., in detail in U.S. Patent Nos. 5,273,760 and 5,286,493.

Other examples of sustained release formulations and coatings which may be used in accordance with the present invention include those described in U.S. Patent Nos. 5,324,351; 5,356,467, and 5,472,712.

### Alkylcellulose Polymers

Cellulosic materials and polymers, including alkylcelluloses, provide hydrophobic materials well suited for coating the beads according to the invention. Simply by way of example, one preferred alkylcellulosic polymer is ethylcellulose, although the artisan will appreciate that other cellulose and/or alkylcellulose polymers may be readily employed, singly or in any combination, as all or part of a hydrophobic coating according to the invention.

One commercially-available aqueous dispersion of ethylcellulose is Aquacoat^{®} (FMC Corp., Philadelphia, Pennsylvania, U.S.A.). Aquacoat^{®} is prepared by dissolving the ethylcellulose in a water-immiscible organic solvent and then emulsifying the same in water in the presence of a surfactant and a stabilizer. After homogenization to generate submicron droplets, the organic solvent is evaporated under vacuum to form a pseudolatex. The plasticizer is not incorporated in the pseudolatex during the manufacturing phase. Thus, prior to using the same as a coating, it is necessary to mix the Aquacoat^{®} with a suitable plasticizer prior to use. Another aqueous dispersion of ethylcellulose is commercially available as Surelease^{®} (Colorcon, Inc., West Point, Pennsylvania, U.S.A.). This product is prepared by incorporating plasticizer into the dispersion during the manufacturing process. A hot melt of a polymer, plasticizer (dibutyl sebacate), and stabilizer (oleic acid) is prepared as a homogeneous mixture, which is then diluted with an alkaline solution to obtain an aqueous dispersion that can be applied directly onto substrates.

### ACRYLIC POLYMERS

In other preferred embodiments of the present invention, the hydrophobic material comprising the sustained release coating is a pharmaceutically acceptable acrylic polymer, including but not limited to acrylic acid and methacrylic acid copolymers, methyl methacrylate copolymers, ethoxyethyl methacrylates, cyanoethyl methacrylate, poly(acrylic acid), poly(methacrylic acid), methacrylic acid alkylamide copolymer, poly(methyl methacrylate), polymethacrylate, poly(methyl methacrylate) copolymer, polyacrylamide, aminoalkyl methacrylate copolymer, poly(methacrylic acid anhydride), and glycidyl methacrylate copolymers.

In certain preferred embodiments, the acrylic polymer is comprised of one or more ammonio methacrylate copolymers. Ammonio methacrylate copolymers are well known in the art, and are described in the National Formulary XVII as fully polymerized copolymers of acrylic and methacrylic acid esters with a low content of quaternary ammonium groups.

In order to obtain a desirable dissolution profile, it may be necessary to incorporate two or more ammonio methacrylate copolymers having differing physical properties, such as different molar ratios of the quaternary ammonium groups to the neutral (meth)acrylic esters.

In certain preferred embodiments, the acrylic coating comprises a mixture of two acrylic resin lacquers commercially available from Rohm Pharma under the Tradenames Eudragit^{®} RL30D and Eudragit^{®} RS30D, respectively. Eudragit^{®} RL30D and Eudragit^{®} RS30D are copolymers of acrylic and methacrylic esters with a low content of quaternary ammonium groups, the molar ratio of ammonium groups to the remaining neutral (meth)acrylic esters being 1:20 in Eudragit^{®} RL30D and 1:40 in Eudragit^{®} RS30D. The mean molecular weight is about 150,000. The code designations RL (high permeability) and RS (low permeability) refer to the permeability properties of these agents. Eudragit^{®} RL/RS mixtures are insoluble in water and in digestive fluids. However, coatings formed from the same are swellable and permeable in aqueous solutions and digestive fluids.

The Eudragit^{®} RL/RS dispersions of the present invention may be mixed together in any desired ratio in order to ultimately obtain a sustained release formulation having a desirable dissolution profile. Desirable sustained release formulations may be obtained, for instance, from a retardant coating derived from 100% Eudragit^{®} RL, or 50% Eudragit^{®} RL and 50% Eudragit^{®} RS, or 10% Eudragit^{®} RL:Eudragit^{®} 90% RS. Of course, one skilled in the art will recognize that other acrylic polymers may also be used, such as, for example, Eudragit^{®} L.

### Plasticizers

In embodiments of the present invention where the coating comprises an aqueous dispersion of a hydrophobic material, the inclusion of an effective amount of a plasticizer in the aqueous dispersion of hydrophobic material may further improve the physical properties of the sustained release coating. For example, because ethylcellulose has a relatively high glass transition temperature and does not form flexible films under normal coating conditions, it is preferable to incorporate a plasticizer into an ethylcellulose coating containing sustained release coating before using the same as a coating material. Generally, the amount of plasticizer included in a coating solution is based on the concentration of the film-former, e.g., most often from about 1 to about 50 percent by weight of the film-former. Concentration of the plasticizer, however, can only be properly determined after careful experimentation with the particular coating solution and method of application.

Examples of suitable plasticizers for ethylcellulose include water insoluble plasticizers such as dibutyl sebacate, diethyl phthalate, triethyl citrate, tributyl citrate, and triacetin. Triethyl citrate is an especially preferred plasticizer for the aqueous dispersions of ethyl cellulose utilized in the present invention.

Examples of suitable plasticizers for the acrylic polymers of the present invention include, but are not limited to citric acid esters such as triethyl citrate NF XVI, tributyl citrate, dibutyl phthalate, and 1,2—propylene glycol. Other plasticizers that have proved to be suitable for enhancing the elasticity of the films formed from acrylic films such as Eudragit^{®} RL/RS lacquer solutions include polyethylene glycols, propylene glycol, diethyl phthalate, castor oil, and triacetin. Triethyl citrate is an especially preferred plasticizer for the aqueous dispersions of acrylic polymers utilized in the present invention.

It has further been found that the addition of a small amount of talc reduces the tendency of the aqueous dispersion to stick during processing, and acts as a polishing agent.

### SUSTAINED RELEASE OSMOTIC DOSAGE

Sustained release dosage forms according to the present invention may also be prepared as osmotic dosage formulations. The osmotic dosage forms preferably include a bilayer core comprising a drug layer (containing the hydrocodone (or hydrocodone salt) and optionally the naltrexone (or naltrexone salt)) and a delivery or push layer (which may contain the naltrexone (or naltrexone salt)), wherein the bilayer core is surrounded by a semipermeable wall and optionally having at least one passageway disposed therein.

The expression "passageway" as used for the purpose of this invention, includes aperture, orifice, bore, pore, or porous element through which hydrocodone or hydrocodone salt (with or without the naltrexone or naltrexone salt) can be pumped, diffuse or migrate through a fiber, capillary tube, porous overlay, porous insert, microporous member, or porous composition. The passageway can also include a compound that erodes or is leached from the wall in the fluid environment of use to produce at least one passageway. Representative compounds for forming a passageway include erodible poly(glycolic) acid, or poly(lactic) acid in the wall; a gelatinous filament; a water-removable poly(vinyl alcohol); leachable compounds such as fluid-removable pore-forming polysaccharides, acids, salts or oxides. A passageway can be formed by leaching a compound from the wall, such as sorbitol, sucrose, lactose, maltose, or fructose, to form a sustained-release dimensional pore-passageway. The passageway can have any shape, such as round, triangular, square and elliptical, for assisting in the sustained metered release of hydrocodone or hydrocodone salt from the dosage form. The dosage form can be manufactured with one or more passageways on one or more surfaces of the dosage form. A passageway and equipment for forming a passageway are disclosed in U.S. Patent Nos. 3,845,770; 3,916,899; 4,063,064 and 4,088,864. Passageways comprising sustained-release dimensions sized, shaped and adapted as a releasing-pore formed by aqueous leaching to provide a releasing-pore of a sustained-release rate are disclosed in U.S. Patent Nos. 4,200,098 and 4,285,987.

In certain embodiments, the bilayer core comprises a drug layer with hydrocodone or a salt thereof and a displacement or push layer containing the naltrexone or a salt thereof. In certain embodiments, the drug layer may also comprise at least one polymer hydrogel. The polymer hydrogel may have an average molecular weight of between about 500 and about 6,000,000. Examples of polymer hydrogels include but are not limited to a maltodextrin polymer comprising the formula (C₆ H₁₂ O₅)ₙ-H₂O, wherein n is 3 to 7,500, and the maltodextrin polymer comprises a 500 to 1,250,000 number-average molecular weight; a poly(alkylene oxide) represented by, e.g., a poly(ethylene oxide) and a poly(propylene oxide) having a 50,000 to 750,000 weight-average molecular weight, and more specifically represented by a poly(ethylene oxide) of at least one of 100,000, 200,000, 300,000 or 400,000 weight-average molecular weights; an alkali carboxyalkylcellulose, wherein the alkali is sodium or potassium, and the alkylcelullose has a 10,000 to 175,000 weight-average molecular weight; and a copolymer of ethylene-acrylic acid, including methacrylic and ethacrylic acid of 10,000 to 500,000 number-average molecular weight.

In certain embodiments of the present invention, the delivery or push layer comprises an osmopolymer. Examples of an osmopolymers include but are not limited to a member selected from the group consisting of a polyalkylene oxide and a carboxyalkylcellulose. The polyalkylene oxide possesses a 1,000,000 to 10,000,000 weight-average molecular weight. The polyalkylene oxide may be a member selected from the group consisting of polymethylene oxide, polyethylene oxide, polypropylene oxide, polyethylene oxide having a 1,000,000 average molecular weight, polyethylene oxide comprising a 5,000,000 average molecular weight, polyethylene oxide comprising a 7,000,000 average molecular weight, cross-linked polymethylene oxide possessing a 1,000,000 average molecular weight, and polypropylene oxide of 1,200,000 average molecular weight. A typical osmopolymer carboxyalkylcellulose comprises a member selected from the group consisting of alkali carboxyalkylcellulose, sodium carboxymethylcellulose, potassium carboxymethylcellulose, sodium carboxyethylcellulose, lithium carboxymethylcellulose, sodium carboxyethylcellulose, and a carboxyalkylhydroxyalkylcellulose such as carboxymethylhydroxyethyl cellulose, carboxyethylhydroxyethylcellulose and carboxymethylhydroxypropylcellulose. The osmopolymers used for the displacement layer exhibit an osmotic pressure gradient across the semipermeable wall. The osmopolymers imbibe fluid into dosage form, thereby swelling and expanding as an osmotic hydrogel (also known as osmogel), whereby they push the hydrocodone or pharmaceutically acceptable salt thereof from the osmotic dosage form.

The push layer may also include one or more osmotically effective compounds also known as osmagents and as osmotically effective solutes. They imbibe an environmental fluid, for example, from the gastrointestinal tract, into the dosage form and contribute to the delivery kinetics of the displacement layer. Examples of osmotically active compounds comprise a member selected from the group consisting of osmotic salts and osmotic carbohydrates. Examples of specific osmagents include but are not limited to sodium chloride, potassium chloride, magnesium sulfate, lithium phosphate, lithium chloride, sodium phosphate, potassium sulfate, sodium sulfate, potassium phosphate, glucose, fructose and maltose.

The push layer may optionally include a hydroxypropylalkylcellulose possessing a 9,000 to 450,000 number-average molecular weight. The hydroxypropylalkylcellulose is represented by a member selected from the group consisting of hydroxypropylmethylcellulose, hydroxypropylethylcellulose, hydroxypropyl isopropyl cellulose, hydroxypropylbutylcellulose, and hydroxypropylpentylcellulose.

The push layer optionally may comprise a nontoxic colorant or dye. Examples of colorants or dyes include but are not limited to Food and Drug Administration Colorant (FD&C), such as FD&C No. 1 blue dye, FD&C No. 4 red dye, red ferric oxide, yellow ferric oxide, titanium dioxide, carbon black, and indigo.

The push layer may also optionally comprise an antioxidant to inhibit the oxidation of ingredients. Some examples of antioxidants include but are not limited to a member selected from the group consisting of ascorbic acid, ascorbyl palmitate, butylated hydroxyanisole, a mixture of 2 and 3 tertiary-butyl-4-hydroxyanisole, butylated hydroxytoluene, sodium isoascorbate, dihydroguaretic acid, potassium sorbate, sodium bisulfate, sodium metabisulfate, sorbic acid, potassium ascorbate, vitamin E, 4-chloro-2,6-ditertiary butylphenol, alphatocopherol, and propylgallate.

In certain alternative embodiments, the dosage form comprises a homogenous core comprising hydrocodone or a pharmaceutically acceptable salt thereof, the naltrexone or pharmaceutically acceptable salt thereof, a pharmaceutically acceptable polymer (e.g., polyethylene oxide), optionally a disintegrant (e.g., polyvinylpyrrolidone), and optionally an absorption enhancer (e.g., a fatty acid, a surfactant, a chelating agent, a bile salt, etc.). The homogenous core is surrounded by a semipermeable wall having a passageway (as defined above) for the release of the hydrocodone or pharmaceutically acceptable salt thereof.

In certain embodiments, the semipermeable wall comprises a member selected from the group consisting of a cellulose ester polymer, a cellulose ether polymer and a cellulose ester-ether polymer. Representative wall polymers comprise a member selected from the group consisting of cellulose acylate, cellulose diacylate, cellulose triacylate, cellulose acetate, cellulose diacetate, cellulose triacetate, mono-, di- and tricellulose alkenylates, and mono-, di- and tricellulose alkinylates. The poly(cellulose) used for the present invention comprises a number-average molecular weight of 20,000 to 7,500,000.

Additional semipermeable polymers include acetaldehyde dimethycellulose acetate, cellulose acetate ethylcarbamate, cellulose acetate methylcarbamate, cellulose diacetate, propylcarbamate, cellulose acetate diethylaminoacetate, semipermeable polyamide, semipermeable polyurethane, semipermeable sulfonated polystyrene, semipermeable cross-linked polymer formed by the coprecipitation of a polyanion and a polycation as described in U.S. Patent Nos. 3,173,876, 3,276,586, 3,541,005, 3,541,006 and 3,546,876, semipermeable polymers as disclosed by Loeb and Sourirajan in U.S. Patent No. 3,133,132, semipermeable crosslinked polystyrenes, semipermeable cross-linked poly(sodium styrene sulfonate), semipermeable crosslinked poly(vinylbenzyltrimethyl ammonium chloride), and semipermeable polymers possessing a fluid permeability of 2.5×10⁻⁸ to 2.5×10⁻² (cm² /hr·atm) expressed per atmosphere of hydrostatic or osmotic pressure difference across the semipermeable wall. Other polymers useful in the present invention are known in the art in U.S. Patent Nos. 3,845,770, 3,916,899 and 4,160,020, and in Handbook of Common Polymers, Scott, J. R. and W. J. Roff, 1971, CRC Press, Cleveland, Ohio.

In certain embodiments, the semipermeable wall is preferably nontoxic, inert, and it maintains its physical and chemical integrity during the dispensing life of the drug.

In certain embodiments, the dosage form comprises a binder. An example of a binder includes, but is not limited to a therapeutically acceptable vinyl polymer having a 5,000 to 350,000 viscosity-average molecular weight, represented by a member selected from the group consisting of poly-n-vinylamide, poly-n-vinylacetamide, poly(vinyl pyrrolidone), also known as poly-n-vinylpyrrolidone, poly-n-vinylcaprolactone, poly-n-vinyl-5-methyl-2-pyrrolidone, and poly-n-vinyl-pyrrolidone copolymers with a member selected from the group consisting of vinyl acetate, vinyl alcohol, vinyl chloride, vinyl fluoride, vinyl butyrate, vinyl laureate, and vinyl stearate. Other binders include for example, acacia, starch, gelatin, and hydroxypropylalkylcellulose of 9,200 to 250,000 average molecular weight.

In certain embodiments, the dosage form comprises a lubricant, which may be used during the manufacture of the dosage form to prevent sticking to the die wall or punch faces. Examples of lubricants include but are not limited to magnesium stearate, sodium stearate, stearic acid, calcium stearate, magnesium oleate, oleic acid, potassium oleate, caprylic acid, sodium stearyl fumarate, and magnesium palmitate.

In certain preferred embodiments, the present invention includes a therapeutic composition comprising 5 to 20 mg of the hydrocodone or pharmaceutically acceptable salt thereof, 25 to 500 mg of poly(alkylene oxide) having a 150,000 to 500,000 average molecular weight, 1 to 50 mg of polyvinylpyrrolidone having a 40,000 average molecular weight, and 0 to about 7.5 mg of a lubricant. The 0.05 to 0.56 mg of naltrexone or pharmaceutically acceptable salt thereof is preferably in the drug layer.

### SUPPOSITORIES

The sustained release formulations of the present invention may be formulated as a pharmaceutical suppository for rectal administration comprising hydrocodone (or hydrocodone salt) and naltrexone (or naltrexone salt) in the dosages disclosed herein. Preparation of sustained release suppository formulations is described in, e.g., U.S. Patent No. 5,215,758.

Prior to absorption, the drug must be in solution. In the case of suppositories, solution must be preceded by dissolution of the base, or the melting of the base and subsequent partition of the drug from the base into the rectal fluid. The absorption of the drug into the body may be altered by the suppository base. Thus, the particular base to be used in conjunction with a particular drug must be chosen giving consideration to the physical properties of the drug. For example, lipid-soluble drugs will not partition readily into the rectal fluid, but drugs that are only slightly soluble in the lipid base will partition readily into the rectal fluid.

Among the different factors affecting the dissolution time (or release rate) of the drugs are the surface area of the drug substance presented to the dissolution solvent medium, the pH of the solution, the solubility of the substance in the specific solvent medium, and the driving forces of the saturation concentration of dissolved materials in the solvent medium. Generally, factors affecting the absorption of drugs from suppositories administered rectally include suppository vehicle, absorption site pH, drug pKa, degree of ionization, and lipid solubility.

The suppository base chosen should be compatible with the active agents(s) of the present invention. Further, the suppository base is preferably non-toxic and nonirritating to mucous membranes, melts or dissolves in rectal fluids, and is stable during storage.

In certain preferred embodiments of the present invention for both water-soluble and water-insoluble drugs, the suppository base comprises a fatty acid wax selected from the group consisting of mono-, di- and triglycerides of saturated, natural fatty acids of the chain length C₁₂ to C₁₈.

In preparing the suppositories of the present invention other excipients may be used. For example, a wax may be used to form the proper shape for administration via the rectal route. This system can also be used without wax, but with the addition of diluent filled in a gelatin capsule for both rectal and oral administration.

Examples of suitable commercially available mono-, di- and triglycerides include saturated natural fatty acids of the 12-18 carbon atom chain sold under the trade name Novata TM (types AB, AB, B,BC, BD, BBC, E, BCF, C, D and 299), manufactured by Henkel, and Witepsol TM (types H5, H12, H15, H175, H185, H19, H32, H35, H39, H42, W25, W31, W35, W45, S55, S58, E75, E76 and E85), manufactured by Dynamit Nobel.

Other pharmaceutically acceptable suppository bases may be substituted in whole or in part for the above-mentioned mono-, di- and triglycerides. The amount of base in the suppository is determined by the size (i.e. actual weight) of the dosage form, the amount of base (e.g., alginate) and active agent used. Generally, the amount of suppository base is from about 20 percent to about 90 percent by weight of the total weight of the suppository. Preferably, the amount of base in the suppository is from about 65 percent to about 80 percent, by weight of the total weight of the suppository.

### OTHER FORMS

The invention disclosed herein is meant to encompass the use of all pharmaceutically acceptable salts thereof of the hydrocodone and naltrexone. The pharmaceutically acceptable salts include, but are not limited to, metal salts such as sodium salt, potassium salt, secium salt and the like; alkaline earth metals such as calcium salt, magnesium salt and the like; organic amine salts such as triethylamine salt, pyridine salt, picoline salt, ethanolamine salt, triethanolamine salt, dicyclohexylamine salt, N,N'-dibenzylethylenediamine salt and the like; inorganic acid salts such as hydrochloride, hydrobromide, sulfate, phosphate and the like; organic acid salts such as formate, acetate, trifluoroacetate, maleate, tartrate, bitartrate and the like; sulfonates such as methanesulfonate, benzenesulfonate, p-toluenesulfonate, and the like; amino acid salts such as arginate, asparginate, glutamate and the like.

The combination of the hydrocodone (or hydrocodone salt) and the naltrexone (or naltrexone salt) can be employed in admixtures with conventional excipients, i.e., pharmaceutically acceptable organic or inorganic carrier substances suitable for oral administration, known to the art in order to provide a sustained release of at least the hydrocodone or salt thereof. Suitable pharmaceutically acceptable carriers include but are not limited to, alcohols, gum arabic, vegetable oils, benzyl alcohols, polyethylene glycols, gelate, carbohydrates such as lactose, amylose or starch, magnesium stearate, talc, silicic acid, viscous paraffin, perfume oil, fatty acid monoglycerides and diglycerides, pentaerythritol fatty acid esters, hydroxymethylcellulose, polyvinylpyrrolidone, etc. The pharmaceutical preparations can be sterilized and if desired, mixed with auxiliary agents, e.g., lubricants, disintegrants, preservatives, stabilizers, wetting agents, emulsifiers, salts for influencing osmotic pressure buffers, coloring, flavoring and/or aromatic substances and the like. The compositions intended for oral use may be prepared according to any method known in the art and such compositions may contain one or more agents selected from the group consisting of inert, non-toxic pharmaceutically acceptable excipients suitable for the manufacture of tablets. Such excipients include, for example, an inert diluent such as lactose; granulating and disintegrating agents such as cornstarch; binding agents such as starch; and lubricating agents such as magnesium stearate. The tablets may be uncoated or they may be coated by known techniques for elegance or to delay release of the active ingredients. Formulations for oral use may also be presented as hard gelatin capsules wherein the active ingredient is mixed with an inert diluent.

The oral dosage forms of the present invention may be in the form of tablets, troches, lozenges, powders or granules, hard or soft capsules, microparticles (e.g., microcapsules, microspheres and the like), buccal tablets, suppositories, etc. The hydrocodone (or hydrocodone salt) and naltrexone (or naltrexone salt) may be substantially interdispersed with one another.

In certain embodiments, the present invention provides a method of deterring parenteral abuse of an oral hydrocodone dosage form (or hydrocodone salt) by preparing any of the hydrocodone/naltrexone dosage forms as disclosed above.

In certain embodiments, the present invention provides a method of deterring diversion of an oral hydrocodone dosage form comprising preparing any of the hydrocodone/naltrexone dosage forms as disclosed above.

In certain embodiments, the present invention provides for a method of treating pain by administering to a human patient a dosage form as described above.

The following examples illustrate various aspects of the present invention. They are not to be construed to limit the claims in any manner whatsoever.

### EXAMPLE 1

Sustained Release Hydrocodone formulations containing naltrexone hydrochloride are prepared in this prophetic example with the formula in Table 1 below:

**TABLE 1**

| **Ingredients** | **Amt/Unit (mg)** | **Amount/Batch (gm)** |
|---|---|---|
| Hydrocodone HCl anhydrous | 20.0 | 209.6* |
| Spray Dried Lactose | 59.85 | 598.5 |
| Povidone | 5.0 | 50.0 |
| Eudragit RS30D (solids) | 10.0 | 100 |
| Triacetin | 2.0 | 20.0 |
| Naltrexone HCI dihydrate | 0.25 | 2.50 |
| Stearyl Alcohol | 25.0 | 250.0 |
| Talc | 2.5 | 25.0 |
| Magnesium Stearate | 1.25 | 12.5 |
| Opadry Pink Y-S-14518A | 5.0 | 50.0 |
| Total | 135.95 | 1368.1 |

| | | |
|---|---|---|
| * adjusted for 99.6% assay and 4.2 % residual moisture. | | |

In this example, the naltrexone hydrochloride is added to the formulation during the granulation process. The process is set forth below:
1. Dispersion: Naltrexone HC1 is dissolved in water and the solution is added to a Eudragit/Triacetin dispersion.
2. Granulation: Spray the Eudragit/Triacetin dispersion onto the Hydrocodone HC1, Spray Dried Lactose and Povidone using a fluid bed granulator.
3. Milling: Discharge the granulation and pass through a mill with approximately 1 mm openings (18 mesh screen).
4. Waxing: Melt the stearyl alcohol at about 50 degrees C and add to the milled granulation using a high shear mixer. Allow to cool at room temperature on trays or a fluid bed.
5. Milling: Pass the cooled granulation through a mill with approximately 18 mesh screen.
6. Lubrication: Lubricate the granulation with talc and magnesium stearate using a mixer.
7. Compression: Compress the granulation into tablets using a Kilian tablet press.
8. Film Coating: Apply an aqueous film coat to the tablets using a rotary pan.

### EXAMPLE 2

Hydrocodone salt /naltrexone salt sustained release osmotic tablets are produced in this prophetic example with the formula set forth in Table 2 below:

**TABLE 2**

| ***Ingredient*** | ***Amt*/*unit (mg)*** |
|---|---|
| *Drug Layer:* | |
| Hydrocodone hydrochloride anhydrous | 20.0 |
| Naltrexone HCL dihydrate | 0.25 |
| Polyethylene oxide | 130.24 |
| Povidone | 8.8 |
| Magnesium Stearate | 1.76 |
| *Displacement Layer:* | |
| Polyethylene oxide | 85.96 |
| Sodium chloride | 40.50 |
| Hydroxypropylmethylcellulose | 6.75 |
| Ferric Oxide | 1.35 |
| Magnesium Stearate | 0.34 |
| BHT | 0.10 |
| *Semipermeable Wall:* | |
| Cellulose acetate | 38.6 |

The dosage form having the above formulation is prepared according to the following procedure:

First, the hydrocodone hydrochloride anhydrous, the naltrexone hydrochloride dihydrate, poly(ethylene oxide) possessing a 200,000 average molecular weight, and polyvinylpyrrolidone having a 40,000 average molecular weight is added to a mixer and mixed for 10 minutes. Then, denatured anhydrous alcohol is added to the blended materials with continuous mixing for 10 minutes. Then, the wet granulation is passed through a 20 mesh screen, allowed to dry at room temperature for 20 hours, and then passed through a 16 mesh screen. Next, the granulation is transferred to the mixer, mixed and lubricated with magnesium stearate.

Then, the displacement or push composition for pushing the hydrocodone HC1/naltrexone HC1 composition from the dosage form is prepared as follows: first 3910 g of hydroxypropylmethylcellulose possessing a 11,200 average molecular weight is dissolved in 45,339 g of water. Then, 101 g of butylated hydroxytoluene is dissolved in 650 g of denatured anhydrous alcohol. Next, 2.5 kg of the hydroxypropylmethylcellulose aqueous solution is added with continuous mixing to the butylated hydroxytoluene alcohol solution. Then, binder solution preparation is completed by adding with continuous mixing the remaining hydroxypropylmethylcellulose aqueous solution to the butylated hydroxytoluene alcohol solution.

Next, 36,000 g of sodium chloride is sized using a Quadro Comil^{®} mill equipped with a 21 mesh screen. Then, 1200 g of ferric oxide is passed through a 40 mesh screen. Then, the screened materials, 76,400 g of pharmaceutically acceptable poly(ethylene oxide) possessing a 7,500,000 average molecular weight, and 2500 g of hydroxypropylmethylcellulose having a 11,200 average molecular weight are added to a Glatt^{®} Fluid Bed Granulation's bowl. The bowl is attached to the granulator and the granulation process is initiated for effecting granulation. Next, the dry powders are air suspended and mixed for 10 minutes. Then, the binder solution is sprayed from 3 nozzles onto the powder. The granulating is monitored during the process as follows: total solution spray rate of 800 g/min; inlet temperature 43°C and air flow 4300 m³/hr. At the end of solution spraying, 45,033 g, the resultant coated granulated particles are subjected to a drying process for 35 minutes.

The coated granules are sized using a Quadro Comil® mill with an 8 mesh screen. The granulation is transferred to a Tote® Tumbler, mixed and lubricated with 281.7 g of magnesium stearate.

Next, the drug composition comprising the hydrocodone HC1/naltrexone HC1 and the push composition are compressed into bilayer tablets on a Kilian® Tablet press. First, the drug composition is added to the die cavity and precompressed, then 135 mg of the push composition is added and the layers are pressed under a pressure head of 3 metric tons into a 11/32 inch (0.873 cm) diameter contacting layer arrangement.

The bilayered arrangements are coated with a semipermeable wall. The wall forming composition comprises 100% cellulose acetate having a 39.8% acetyl content. The wall-forming composition is dissolved in acetone:water (95:5 wt:wt) cosolvent to make a 4% solid solution. The wall-forming composition is sprayed onto and around the bilayers in a 24 inch (60 cm) Vector® Hi-Coater. Next, one 20 mil (0.508 mm) exit passageway is drilled through the semipermeable wall to connect the drug hydrocodone layer with the exterior of the dosage form. The residual solvent is removed by drying for 72 hours at 45°C and 45% humidity. Next, the osmotic dosage systems are dried for 4 hours at 45°C to remove excess moisture.

### EXAMPLE 3

Hydrocodone 5 mg/naltrexone 0.0625 mg sustained release capsules are prepared in this prophetic example with the formula set forth in Table 3 below:

**Table 3**

| *Ingredient* | *Amt*/*unit* (*mg*) |
|---|---|
| Hydrocodone HCl anhydrous | 5.0 |
| Naltrexone HCl dihydrate | 0.0625 |
| Stearic Acid | 8.15 |
| Stearic Alcohol | 24.00 |
| Eudragit RSPO | 82.79 |
| Total | 120 |

The formulation above is prepared according to the following procedure:
1. Pass the stearyl alcohol flakes through an impact mill.
2. Blend the Hydrocodone HC1, Naltrexone HC1, stearic acid, stearyl alcohol and the Eudragit RSPO in a suitable blender/mixer.
3. Continuously feed the blended material into a twin screw extruder at elevated temperatures, and collect the resultant strands on a conveyor.
4. Allow the strands to cool on the conveyor.
5. Cut the strands into 1 mm pellets using a pelletizer.
6. Screen the pellets for fines and oversized pellets to an acceptable range of about 0.8 - 1.4 mm in size.
7. Fill into capsules with a fill weight of 120 mg/capsule (fill into size 2 capsules).

### EXAMPLE 4

Hydrocodone 5 mg/naltrexone 0.0625 mg sustained release capsules are prepared in this prophetic example according to the following procedure:

Initially, immediate release hydrocodone beads are prepared with the formula set forth in Table 4 below:

**Table 4**

| **Ingredients** | **Amount/Unit (mg)** |
|---|---|
| Hydrocodone HC1 anhydrous | 5.0 |
| Opadry® Clear YS-1-19025A | 1.25 |
| NuPareil (Sugar beads) 30/35 mesh | 59.35 |
| Opadry® Butterscotch YS-1-17307A | 1.90 |
| Total | 62.5 |

### Process

1. Drug layering solution: Dissolve hydrocodone HC1 and Opadry Clear in water.
2. Drug loading: Spray the drug solution onto NuPareil beads in a fluid bed dryer.
3. Coating: Disperse Opadry Butterscotch in water. Spray onto the drug loaded beads.

Sustained Release Beads are then prepared with the formula set forth in Table 5 below:

**Table 5**

| **Ingredients** | **Amount/Unit (mg)** |
|---|---|
| Hydrocodone IR Beads | 53.08 |
| (5mg/62.5mg) | |
| Eudragit®RS 30 D (solids) | 5.04 |
| Eudragit®RL 30 D (solids) | 0.27 |
| Triethyl Citrate | 1.05 |
| Cab-O-Sil® | 0.27 |
| Opadry® Clear YS-1-19025A | 2.79 |
| Total | 62.5 |

### Process

1. Controlled release coating solution: Homogenize triethyl citrate in water. Add the dispersion to Eudragit®RS 30 D and Eudragit®RL 30 D, then add Cab-O-Sil® to mixture.
2. Seal coat solution: Dissolve Opadry® Clear in water.
3. Coating: Apply the control release coating solution, followed by the seal coat solution onto Hydrocodone HC1 IR beads using a fluidized bed bottom-spray technique.
4. Curing: Place the coated beads on tray and cure in oven for 24 hours at 45°C.

To develop Hydrocodone/Naltrexone sustained release beads, 0.0625mg of Naltrexone per unit can be included in the above formulation. It can be dissolved together with the Hydrocodone HC1 in the purified water before being sprayed onto the NuPareil beads.

### EXAMPLE 5

In Example 5, a single center, placebo controlled, double-blind, randomized 9-treatment, 3 period crossover trial with an open-label screening phase was conducted. The trial was done to assess the effect of concurrent doses of oral naltrexone (NTX) on the agonist effects of oral immediate-release hydrocodone (HYIR) on minute ventilation in normal, healthy, adult male and female volunteers between 18 to 45 years of age, inclusive, with a body weight ranging from approximately 45 to 100 kg and within 15% of optimum weight.

The study consisted of a screening phase of up to 14 days, an open-label HYIR titration phase of up to 5 days, a double-blind phase that included 3 treatment periods of 1 day with a 24-hour washout period between each treatment period, and an end-of-study visit up to 14 days after the last treatment period. Total duration in the study was at least 39 days.

Prior to enrollment, each subject was qualified for participation in the study using inclusion and exclusion criteria. A detailed medical history was obtained from each subject. The following screening procedures were completed by all subjects prior to starting the open-label HYIR titration phase: physical examination; ECG measurement; vital signs; and clinical laboratory testing (hematology, chemistry, urinalysis, HIV screen, hepatitis screen, drug screen, blood alcohol test, and pregnancy test).

After meeting entry criteria, subjects participated in the open-label HYIR titration phase which was designed to determine the highest tolerated dose of HYIR that produced a detectable change in respiratory drive with minimal adverse effects. The highest tolerable dose of HYIR that produced a detectable change in respiratory drive, defined as an increase from predose of at least 3 Torr in PETCO₂ (End-tidal carbon dioxide concentration (in Torr)) at a MV (minute ventilation) of 20 L/min at 60 and 90 or 90 and 120 minutes postdose, was chosen as the HYIR dose for that subject that was administered in the double-blind portion of the study. Subjects were trained to operate the spirometer used in the CO₂ rebreathing test. Each subject then received 15, 20, or 25 mg of HYIR in ascending doses in up to 3 separate titration sessions with a 24 hour washout between titration sessions. Subjects continued in the open-label phase until they reached the 25-mg HYIR dose without intolerable adverse effects or to a dose with intolerable adverse effects. If the subjects went to an HYIR dose with intolerable adverse effects, the highest dose of HYIR without intolerable adverse effects was used in the double-blind phase. The CO₂ rebreathing test administered during each titration session yielded MV and PETCO₂ values at 30 minutes prior to treatment (0 h) and at 30, 60, 90, 120, and 180 minutes postdose. Those subjects with this change in MV were permitted to continue to the double-blind phase of the study.

Thirty-three (33) subjects enrolled in screening phase. Thirteen (13) withdrew due to lack of respiratory opioid sensitivity or opioid intolerance. Twenty (20) of the subjects were randomized onto double-blind phase and eighteen (18) subjects completed the double-blind phase.

The study medication, mode of administration, dosage forms, unit strengths, and the test treatments and reference treatment for the double-blind phase were as follows:

| Study Medication | Mode | Dosage Form | Unit Strength |
|---|---|---|---|
| 1. Hydrocodone bitartrate(HYIR) | Oral | Tablet | 5mg |
| 2. Naltrexone HCI (NTX) | Oral | Solution | 0.125, 0.25, 0.375, |
| | | | 0.5, 0.75, 1.5, 3.0, and |
| | | | 8.0mg/10mL |
| 3. NTX Solution (NOS) placebo | Oral | Solution | 0.2mg/10mL |

### Test Treatments

HYIR (15, 20, or 25mg; 3, 4, or 5 x 5-mg tablets) + NOS (Naltrexone Oral Solution) placebo
HYIR (15, 20, or 25mg; 3, 4, or 5 x 5-mg tablets) + 0.125 mg NOS
HYIR (15, 20, or 25mg; 3, 4, or 5 x 5-mg tablets) + 0.25 mg NOS
HYIR (15, 20, or 25mg; 3, 4, or 5 x 5-mg tablets) + 0.375 mg NOS
HYTR (15, 20, or 25mg; 3, 4, or 5 x 5-mg tablets) + 0.5 mg NOS
HYIR (15, 20, or 25mg; 3, 4, or 5 x 5-mg tablets) + 0.75 mg NOS
HYIR (15, 20, or 25mg; 3, 4, or 5 x 5-mg tablets) + 1.5 mg NOS
HYIR (15, 20, or 25mg; 3, 4, or 5 x 5-mg tablets) + 3.0 mg NOS
HYIR (15, 20, or 25mg; 3, 4, or 5 x 5-mg tablets) + 8.0 mg NOS

### Reference Treatment

HYIR (15, 20, or 25mg; 3, 4, or 5 x 5-mg tablets) + NOS placebo

HYIR 5-mg tablets were supplied by AAI Pharma, Wilmington, NC.

Naltrexone hydrochloride powder (Mallinckrodt Chemical Inc., St. Louis MO) was used to formulate the NOS. the requires amount of naltrexone powder was weighes out and dissolved separately in 50 ml of distilled water and 50 ml simple syrup, NF for a final volume of 100 ml. These concentrations allowed the same volume (10 ml) of NOS to be administered during each treatment period.

NOS placebo contained a bittering agent; Bitterguard (denatonium benzoate, NF) powder. NOS placebo was prepared using the same vehicle as was used in the preparation of NOS. The appearance and the taste of the placebo solution were similar to the active solution.. The administered volume (10 ml) of NOS placebo was matched to the administered volume (10 ml) of active NOS.

In the double-blind phase, subjects received the effective dose of HYIR determined in the open-label-phase (15, 20, or 25 mg) and 3 of the 9 possible NOS (naltrexone oral solution) treatments (placebo, 0.125, 0.25, 0.375, 0.5, 0.75, 1.5, 3.0, or 8.0 mg) in a 3-period, crossover clinical trial. The HYIR and NOS were administered to each subject following a 6-hour fast. The fast continued through 3 hours postdose. The CO₂ rebreathing test was conducted at least 30 minutes before administration of study medication (0 h) and at 30, 60, 90, 120, and 180 minutes postdose.

### Criteria for Evaluation

Pharmacodynamic: The results of the CO₂ rebreathing test were used to measure the effect of HYIR and HYIR plus NOS on minute ventilation.

Safety: Safety was assessed using adverse events, clinical laboratory results, vital signs, physical examinations, and electrocardiogram (ECG) measurements.

### Statistical Methods

The pharmacodynamic variables derived from a plot of MV versus PETCO₂ included the PETCO₂ at MV rates of 20 and 30 L/min (20-and 30-liter intercept values) and the slope of the MV/PETCO₂ regression line. The maximum change from predose (maximum possible effect, MPE) was calculated for each variable (MPE₂₀, MPE₃₀, and MPEₛₗₒₚₑ) in the open-label (MPE_{(OL)} = maximal respiratory depression) and in the double-blind (MPE_{(DB)} = respiratory depression due to HYIR + NTX) phases of the study. The percent maximal respiratory depression (%MPE) was calculated for each variable with each treatment in the double-blind phase from the ratio of the MPE_{(DB)}/WE_{(OL)} × 100.

The primary pharmacodynamic variables were the %MPE for the 20- and 30-liter intercepts (%MPE₂₀ and %MPE₃₀, respectively) for each treatment in the double-blind phase. Secondary pharmacodynamic variables included the slope of the %MPE and the double-blind phase MPE₂₀, MPE₃₀, and MPEₛₗₒₚₑ. These values were summarized by treatment group using descriptive statistics and were analyzed using mixed effects analysis of variance (ANOVA) models with parameters for random subject, fixed period, and fixed treatment. In addition, the dose-response relationships between NOS dose and %MPE₂₀ and %MPE₃₀ were investigated using a linear contrast test.

### Results

### Pharmacodynamic:

Effect size analysis of the open-label 20- and 30-liter intercept MPE and slope values showed the most sensitive (least variation about the mean) measures of respiratory depression were the 20- and 30-liter intercept MPE values.

Increasing doses of NTX resulted in a statistically significant trend toward less respiratory depression, across all treatments, in the %MPE₂₀, %MPE₃₀, and a double-blind MPE₂₀ and MPE₃₀ values derived from the CO₂ rebreathing test. These data suggest a dose-dependent antagonism of HYIR-induced respiratory depression.

### Safety:

There were no new safety concerns identified with the 15-, 20-, or 25-mg doses of HYIR used to produce respiratory depression or when combined with NTX doses ranging from 0.125 to 8.0 mg.

### Conclusion

Oral NTX, in the range of 0.125 to 8.0 mg, in a dose-dependent manner, blocked respiratory depression induced by 15, 20, or 25 mg of HYIR. There were no new or unexpected safety concerns.

### EXAMPLE 6

Example 6 consisted of open-label and double-blind treatment phases conducted in male and female subjects receiving daily oral methadone maintenance doses from 60 to 90 mg. The methadone maintenance dose was given to the subject the day before each scheduled period. Administration of study medication occurred no sooner than 16 hours and no later than 22 hours after the methadone maintenance dose was given. 14 subjects were enrolled in the study (2 subjects, open-label phase (ascending dose naltrexone safety assessment) and 12 subjects, double-blind phase).

### Open-Label Phase

The open-label phase was a safety assessment of the 2 naltrexone doses (0.75 and 2.0 mg) planned in the protocol in subjects on methadone maintenance therapy. This phase of the study consisted of a screening visit conducted up to 14 days before administration of study drug, and a naltrexone titration visit. During the naltrexone titration visit, 2 subjects were to receive 30 mg of hydrocodone and 0.125 mg of naltrexone at 0 hr, with additional doses of naltrexone, up to a cumulative dose of 2.0 mg, administered at hourly intervals over the next 4 hours. In the open-label phase, neither subject received more than a 1.0-mg cumulative dose of naltrexone before rescue with methadone was required. As a result of the intensity of precipitated withdrawal observed in these 2 subjects, the doses of naltrexone used in the study were changed from placebo, 0.75mg, and 2.0mg to placebo, 0.25mg, and 0.5mg.

### Double-Blind Phase

The double-blind phase was designed as a randomized, 3-period, 3-way crossover, with randomized naltrexone doses and a naltrexone placebo treatment. This phase of the study consisted of a screening visit, conducted up to 14 days before randomization to a specific treatment sequence, and 3 subsequent visits at which double-blind study drug was administered.

Each treatment sequence consisted of 3 periods of 4 hours duration separated by at least a 48-hour washout period. In each period, each subject received a 30-mg dose of hydrocodone plus 1 of 3 different doses ofnaltrexone (placebo, 0.25 mg, or 0.5 mg). The total duration each subject participated in the double-blind phase was approximately 20 days.

After 8 subjects had completed the study, the 0.5-mg dose of naltrexone was dropped from the study. The remaining 4 subjects were enrolled and completed the study receiving only 2 naltrexone doses, placebo and 0.25-mg naltrexone. The original randomization schedule and treatment sequences continued to be used, but the 0.5-mg naltrexone period was dropped from the treatment sequence. The duration of participation in the study for the subjects enrolled after removal of the 0.5-mg dose was approximately 17 days.

The study design was appropriate to assess the time course and magnitude of the effects of 30 mg of hydrocodone given orally in combination with 0.25 and 0.5 mg naltrexone oral doses on several subjective and objective measures in subjects receiving methadone maintenance therapy. This conclusion is based upon the following study design features:

Study bias was controlled through the study design as 2 (3x3) Latin squares (though the 0.5 mg naltrexone treatment was terminated in certain subjects), double-blind administration of study drug and randomized naltrexone dose.

The open-label phase allowed the selection of naltrexone doses that could be tolerated by this subject population. As a result of the intensity of precipitated withdrawal observed in the open-label phase of the study, the doses of naltrexone used in the study were reduced from 0.75 and 2.0 mg to 0.25 and 0.5 mg.

The dependency/addiction of the subject was verified using the Addiction Severity Index.

The pharmacodynamic variables measured the known physiological and subjective effects of opioids.

The physiologic pharmacodynamic variables were measurements of skin temperature and pupil diameter. Opioid agonists are known to produce peripheral arteriolar and venous dilatation and to constrict the pupil due to an excitatory action on the parasympathetic nerve innervating the pupil.

The subjective and objective pharmacodynamic variables in this study included the Subjective and Observer Drug Effect Scales, measures of opioid drug abuse potential and dependence; Subjective and Observer Symptom Rating Scales, recognized measures to monitor opioid withdrawal and maintenance in opioid-dependent individuals; the Street Value Estimation Questionnaire, a subjective measure of abuse potential in opioid-dependent individuals; and the Drug Identification Questionnaire, a questionnaire designed to measure drug discrimination and abuse potential.

The safety parameters in this study were adverse events, clinical laboratory tests, electrocardiograms, and vital signs.

The control treatment in this study was 30 mg hydrocodone plus naltrexone placebo.

Each subject was to receive his or her daily methadone dose at the end of each period. However, if a subject experienced withdrawal that was intolerable, the subject could be given his or her usual dose of methadone as a rescue medication at any time during the period. The 30-mg dose of hydrocodone administered in the periods of this study was equivalent to the 60- to 90-mg oral maintenance dose of methadone.

Subjects enrolled in both the open-label and double-blind phases of the study were to be receiving a daily oral methadone maintenance dose of between 60 to 90 mg, inclusive, and consequently were expected to be physically dependent on opioids.

Measurement of the pharmacodynamic parameters that included pupil diameter, skin temperature, Subjective and Observer Drug Effect Scales, and Subjective and Observer Symptom Rating Scales were made within 0.5 hours prior to each test treatment administration (predose) and at 0.25, 0.5, 1, 2, 3, and 4 hours postdose. The Street Value Estimation Questionnaire was completed at 0.25, 0.5 1, 2, 3, and 4 hours postdose. The Drug Identification Questionnaire was completed at 1 and 3 hours postdose.

The safety measures that included physical examinations, clinical laboratory tests (hematology and blood chemistries), and an ECG were performed at the screening visit and at the end of study or early termination visit. Vital signs and oxygen saturation were recorded at screening, predose and 0.25, 0.5 1, 2, 3, 4 hours postdose, and at end of study. Adverse events were collected from the first day of study drug administration through the release of each subject from the study.

### Pharmacodynamic Measures

The pharmacodynamic measures used in this study are described below.

### Drug Effects Scale (Subjective and Observer)

The Subjective Drug Effects Scale evaluated 4 experiences that the subjects might have had with the different test treatments.
Like this feeling
Bad effects
Feeling sick
Good effect

The subjects were asked to rank on a categorical visual analog scale of 0 to 10 how they felt with respect to the 4 experiences. Higher scores reflected increased opioid agonist effects (euphoria), while a lower score was indicative of decreased opioid agonist effects or an increase in antagonist activity (withdrawal).

The Observer Drug Effects Scale evaluated 4 experiences the subject may have displayed with the different test treatments.
Enjoyment
Dysphoric
III
Euphoria

The observer was asked to rank on a categorical visual analog scale of 0 to 10 how they perceived that the subjects felt for each of the 4 experiences. Higher scores reflected increased opioid agonist effects (euphoria), while a lower score was indicative of decreased opioid agonist effects or an increase in antagonist activity (withdrawal).

### Symptom Rating Scale (Subjective and Observer)

The Subjective Symptom Rating Scale was used by the subject to evaluate symptoms of opioid receptor activity or precipitated withdrawal, in the case of the antagonist items and their intensity level.

| Agonist items | Antagonist items |
|---|---|
| Talkative | Restlessness |
| Energetic | Sick to stomach |
| Heavy/sluggish | Irritable |
| Carefree | Tense |
| Itchy skin | Jittery |
| Happy | Hot or cold flashes |
| Nervous | Skin clammy or damp |
| Content | Face blushing |
| Head nodding | Yawning |
| Relaxed | Watery eyes |
| Pleasant | Runny nose |
| Drifting | Chills/goose flesh |
| Sweating | |

The symptoms were rated on a scale of 1-3:
I don't feel this way at all.
I feel like this somewhat
I really feel this way.

Higher scores reflected an increase in opioid agonist or antagonist symptoms, while a lower score was indicative of a decrease in opioid agonist or antagonist symptoms.

The Observer Symptom Rating Scale Questionnaire was used to evaluate possible signs of opioid receptor agonist and antagonist activity displayed by a subject and their intensity level.

| Agonist items | Antagonist items |
|---|---|
| Itching | Yawning |
| Sluggish | Lacrimating |
| Runny nose | |
| Restlessness | |

The symptoms were rated on a scale of 1-4:
None at all.
Relatively unnoticeable but perceivable on close observation.
Fairly obvious. Don't need to look closely to observe.
Very obvious. Is a persistent feature or appears bothersome to the subject.

Higher scores reflected an increase in opioid agonist or antagonist signs, while a lower score was indicative of a decrease in opioid agonist or antagonist signs.

### Pupil Diameter

The subject's eye was photographed in constant ambient light using a Polaroid (Cambridge, MA) camera fitted with 2X. Pupil diameter from each photo was measured in millimeters using calipers. The same eye was used for all determinations in each period. The eye used for the measurement was documented.

### Drug Identification Questionnaire

The Drug Identification Questionnaire consisted of a list of 10 drug categories using language that would be familiar in the opioid-abusing population. Subjects selected the category to which the test drug was most similar. The following categories were listed on the questionnaire.
Blank or placebo
Opiates (like: morphine, heroin, codeine, methadone)
Opiate antagonist (like: naloxone, naltrexone)
Antipsychotic or neuoleptic (like: haldol, stelazine)
Barbiturates and sleeping medications, (like: quaaludes, pentobarbital, seconal) Antidepressant (like: elavil, imipramine)
PCP or hallucinogens (like: LSD, mescaline, MDA, STP)
Benzodiazepine (like: valium, Librium, ativan, xanax)
Cocaine or stimulants (like: amphetamine, dexedrine, ritalin) Other

### Street Value Estimation

The subject was asked the question, "How much would you pay for this drug on the street?"

The subject would then record directly on the CRF how much they thought the drug was worth.

### Skin Temperature

Skin temperature was measured using a dual-channel, dual-display, electronic thermometer with disposable temperature probes. Temperature was recorded in degrees Celsius.

### Adverse Events

An adverse event was any unfavorable and unintended sign (including abnormal laboratory findings), symptom, or disease temporally associated with the use of a medicinal product, whether or not considered related to the medicinal product. All adverse events, whether spontaneously reported or observed by the investigator, that occurred after administration of the first dose of study medication and up to release from the study were reported on the adverse even form. When adverse events were encountered that required medical intervention, appropriate supportive and/or definitive therapy was provided by appropriately qualified and licensed medical personnel.

### Overall Conclusions

This study was designed to characterized the effect of a range of oral doses of NTX administered in combination with a 30-mg oral dose of HYIR on various subjective and physiologic measures of opioid agonist and antagonist activity in subjects receiving methadone maintenance therapy.

The 30-mg oral dose of HYIR did not produce significant subjective or physiologic opioid agonist activity in this subject population. Following the HYIR plus NTX placebo treatment, there were minimal changes from predose in all pharmacodynamic variables. A rescue dose of methadone was required by 4 of the 12 subjects who received this treatment; an indication of potentially emerging precipitated abstinence.

The primary pharmacodynamic variables in the study were the mean PDmax (maximum predose scores) values for the queries "Like this feeling," "Good effects," "Bad effects," and "Feeling sick." There was a dose-related effect of NTX on the mean PDmax values for all 4 queries on the Subjective Drug Effects Scale. Increasing the dose of NTX from 0.25 to 0.5mg resulted in progressively more negative maximum changes from the predose scores for each query, which in all cases indicated a NTX dose-related antagonism of opioid agonist effects. There were statistically significant differences between the NTX placebo treatment and the 0.25-mg NTX dose for the queries "Liking this feeling" and "Bad effects," and with the 0.5-mg NTX dose for all queries except "Feeling sick."

There was a dose-related effect of NTX, although not always statistically significant, on all secondary pharmacodynamic variables except the agonist total scores from the Subjective and Observer Symptom Rating Scales. The 0.25-mg NTX dose was a threshold dose with a trend toward negative feeling states (decreased opioid agonist effects) and increased antagonist activity (precipitated withdrawal). The 0.5-mg NTX dose produced strong evidence of precipitated withdrawal with statistically significant differences from the NTX placebo treatment in the Subjective and Observer Drug Effects Scales, the antagonist total score from the Subjective and Observer Symptom Rating Scale, and pupil diameter. Approximately 60% and 90% of the subjects receiving the 0.25- and 0.5-mg NTX doses, respectively, required a rescue dose of methadone.

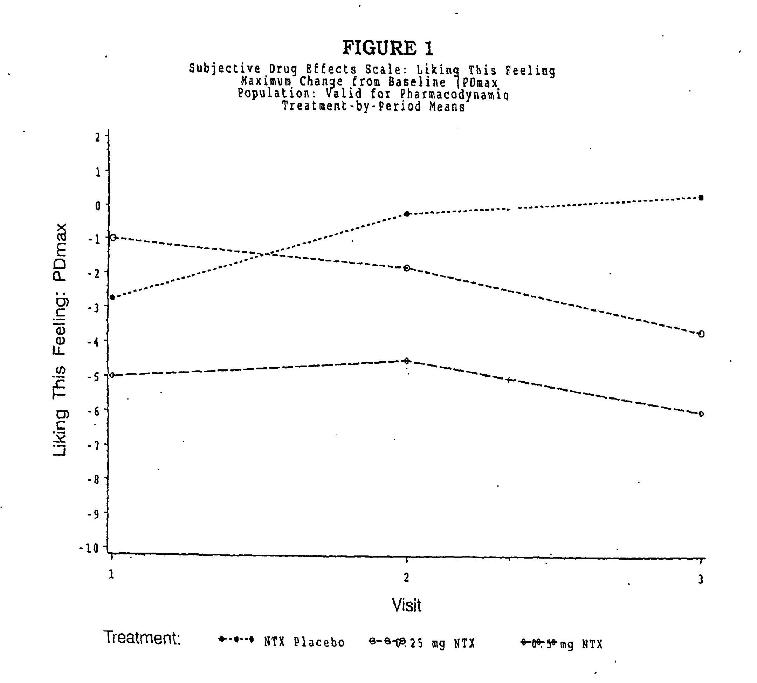

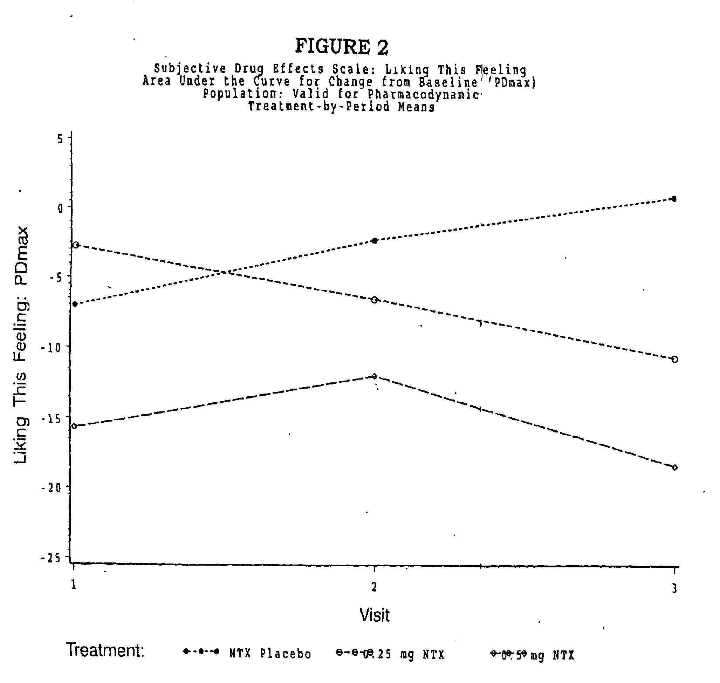

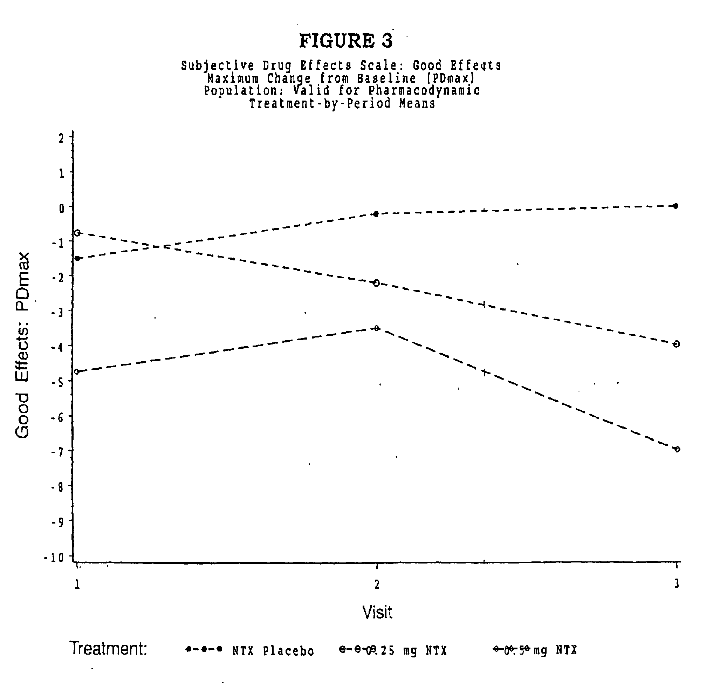

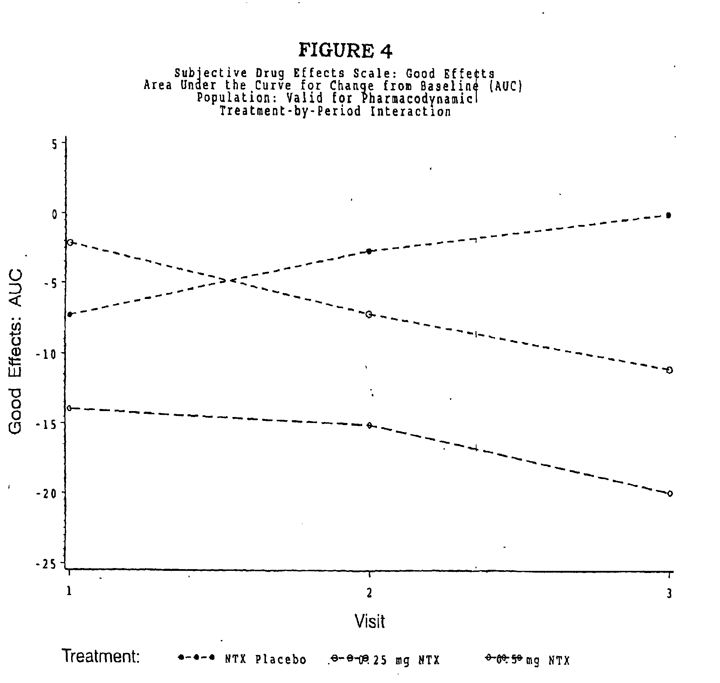

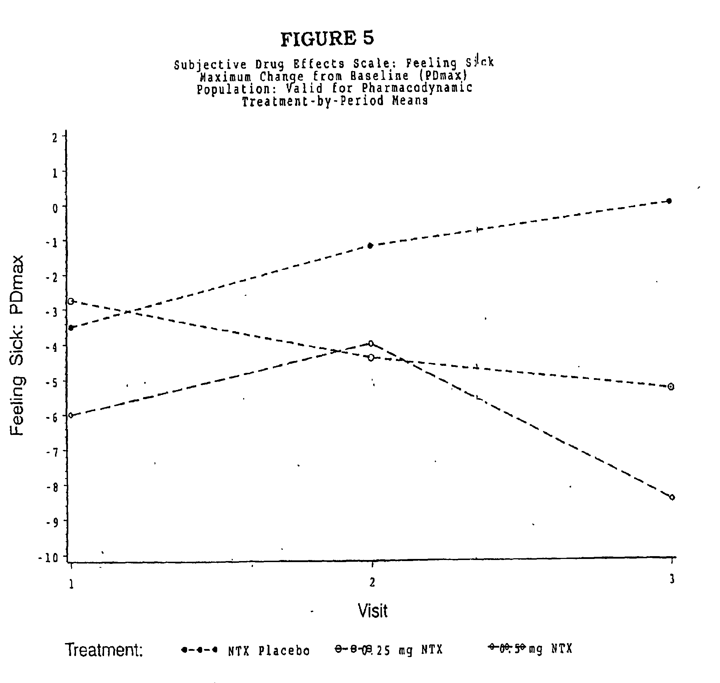

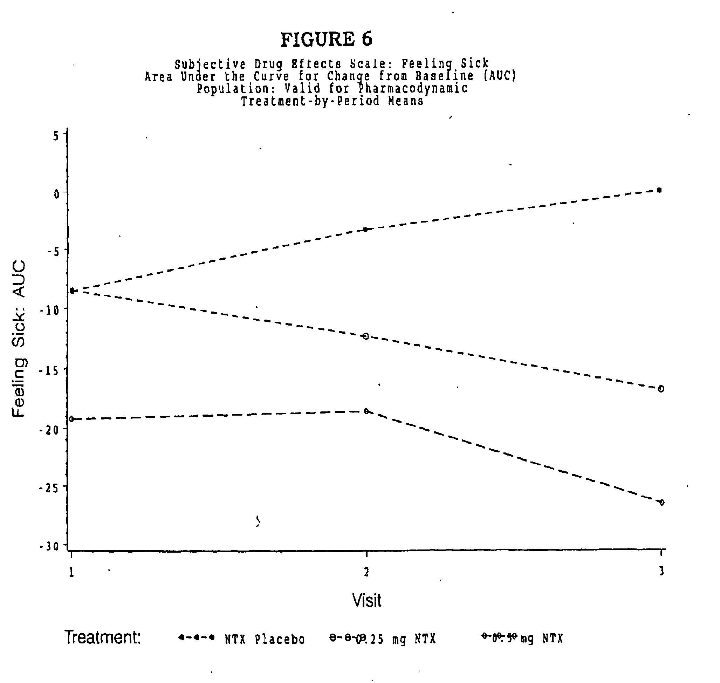

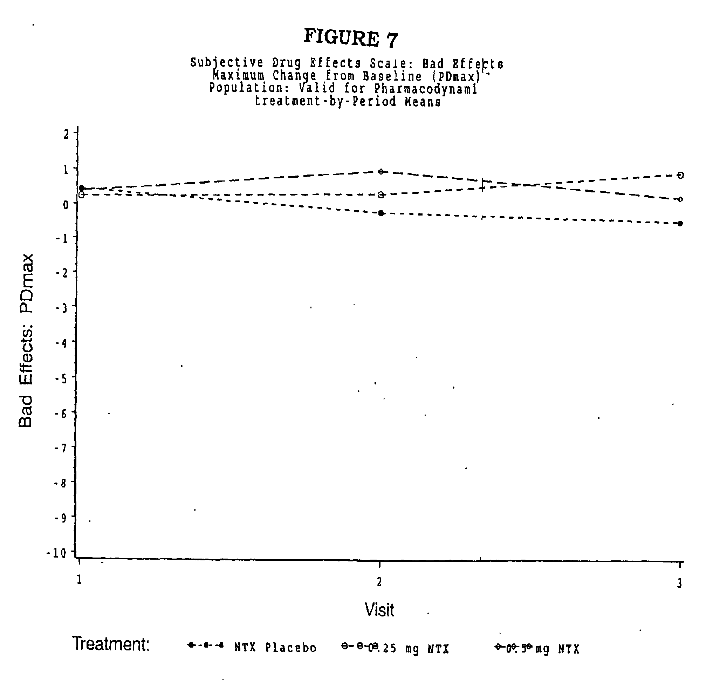

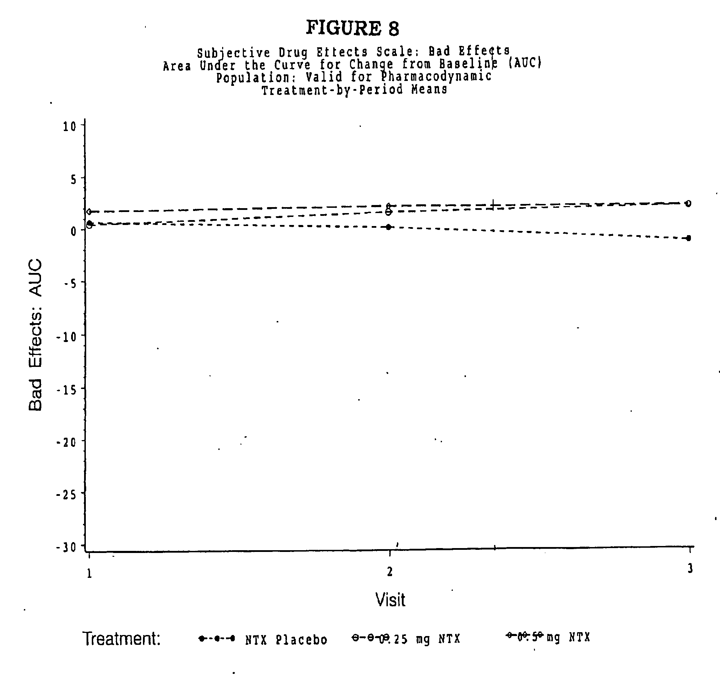

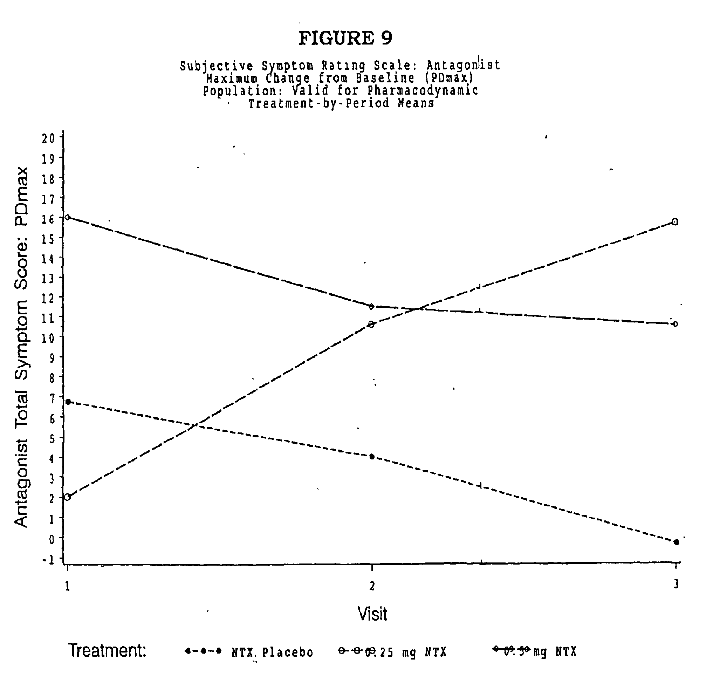

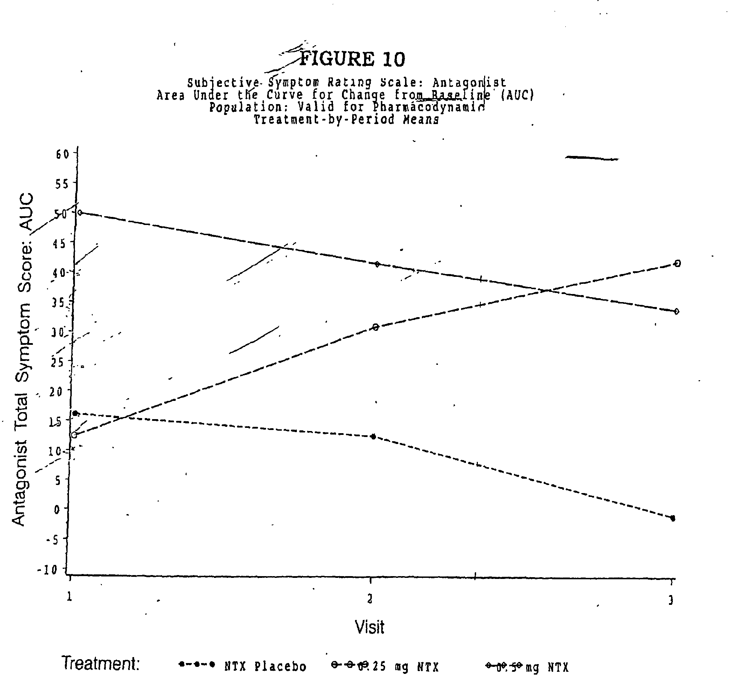

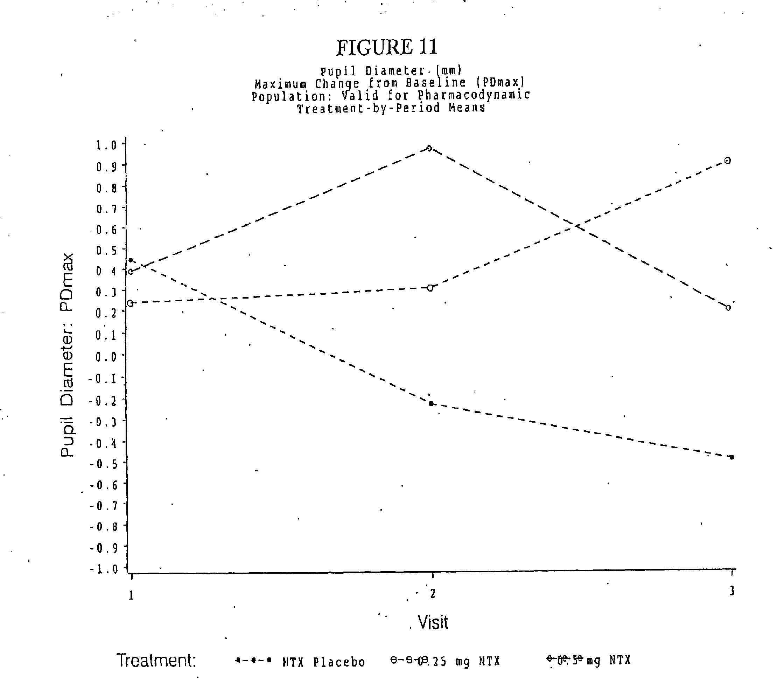

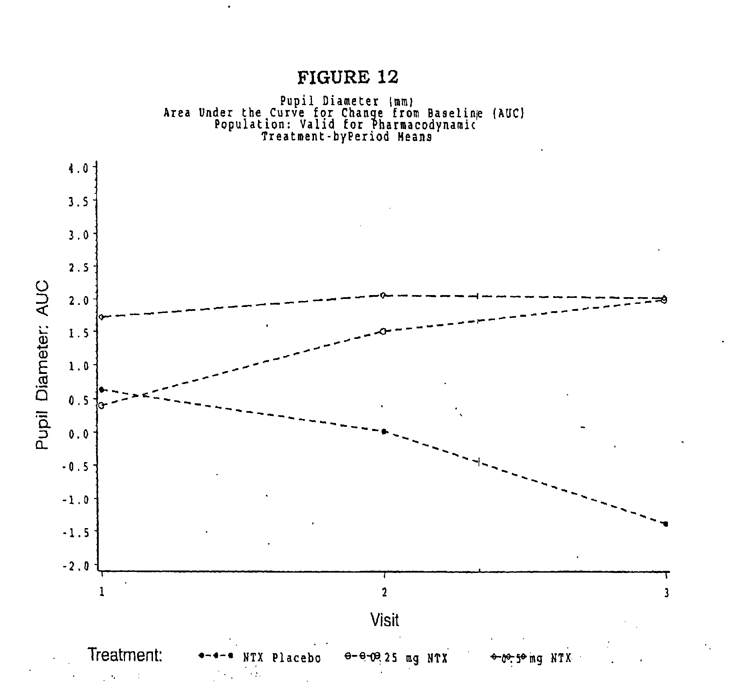

The figures of treatment means for each of the 3 periods (Figures 1-12) for PDmax and AUC for a number of measures of opioid drug showed a trend for changes over the periods that differed for the HYIR + NTX placebo treatment and the HYIR + 0.25 mg NTX and HYIR + 0.5 mg NTX treatments. The trends for the observed means are consistent with the hypothesis of an increase in subjective and physiologic opioid agonist effects over the 3 periods following the administration of HYIR + NTX placebo treatment and an increase in opioid antagonist effect over the 3 periods following the administration of either the HYIR + 0.25 mg NTX or HYIR + 0.5 mg NTX treatments.

Measures of the safety of the coadministration of the 0.25- or 0.5-mg oral dose of NTX with the 30-mg HYIR dose did not suggest any new or unexpected safety concerns in this subject population. There was an increase in the number of treatment-emergent adverse events commonly associated with opioid withdrawal per subject with increasing NTX dose, although most of the treatment-emergent adverse events were mild or moderate. There was only 1 subject with clinically notable abnormal laboratory values and those were attributable to conditions listed in the subject's prior medical history. The occurrence of clinically notable vital sign abnormalities was an isolated event both with respect to subject and treatment.

In conclusion, both the 0.25- and 0.5-mg oral NTX doses were found to elicit aversive effects in methadone-maintained subjects. NTX produced dose-dependent increases in negative feeling states and precipitated withdrawal. The combination of oral NTX (0.25 or 0.5 mg) and oral HYIR (30 mg) did not result in new or unexpected safety concerns. In effect, the addition of low dose NTX to HYIR decreased the appeal, and therefore, the abuse liability potential of HYIR in subjects physically dependent on opioids.

### EXAMPLE 7

Example 7 consisted of a single center trial conducted as a placebo-controlled, double-blind, randomized, 4-treatment, 4-period crossover study, which included a single-blind phase. Each treatment sequence consisted of 4 treatment periods, each of 4 hours duration, separated by at least a 5-day washout interval. In each treatment period, each subject received 15 mg of HYIR orally and either placebo, 0.25, 0.5, or 1.0-mg of NTX. The total duration of this study, including screening, was approximately 52 days.

### Screening Phase

The screening phase was conducted up to 21 days before randomization into the double-blind portion of the study. The subjects participated in a training session for Thermal Discomfort testing. This training involved sequentially applying copper masses heated to 43°C, 46°C and 49°C to a designated site on the forearm for no more than 5 seconds. After each application, subjects assessed pain intensity using the 100-mm visual analog scale (VAS). The procedure was repeated at new skin sites until subjects were able to produce, at the discretion of the investigator, consistently reliable VAS scores. Subjects who were unable to satisfactorily complete the screening phase were discontinued from the study.

### Single-blind Phase

After completion of the screening phase evaluation of Thermal Discomfort was conducted in the single-blind phase as follows:

A site was selected on the forearm and marked with a washable marker. Baseline vital signs were taken. A topical anesthetic (EMLA® cream, AstraZeneca, Wilmington, DE) was applied to the predetermined site on the forearm. At approximately 1.5 hours (after allowing for the anesthetic to take effect) the cream was removed and a thermal stimulus using a copper mass heated to 52°C was applied to the site on the forearm for 3 minutes. Approximately 1 hour was allowed for sensory recovery from the topical anesthetic. Each subject was then given orally 2 placebo 7.5-mg HYIR tablets and 2 placebo NTX tablets.

At 1.5 hours postdosing, vital signs were taken and a Thermal Discomfort test was administered. The Thermal Discomfort testing consisted of sequentially applying a copper mass heated to 43°C, 46°C, and 49°C to the site for 5 seconds. After each application, the subject assessed pain intensity using a 100-mm VAS scale. The VAS scores obtained from these measures were summed and only those subjects who had a summed score of 60 mm or greater were permitted to continue in the screening process.

Those subjects continuing with screening received 2 x 7.5-mg HYIR tablets and 2 placebo NTX tablets. Test measurements were repeated at 1.5 hours after the dose of HYIR for each of the 3 temperatures. Subjects who achieved at least a 20-mm decrease in the summed VAS pain score from the previous testing session were eligible for inclusion into the double-blind portion of the study.

### Double-blind Phase

After at least 5 days, those subjects who successfully completed the single-blind phase were randomized into the double-blind phase of the study. Healthy male and female volunteers were enrolled in this study. Each subject participated in 4 treatment periods in a crossover design and received 15 mg HYIR with each of 4 NTX doses (placebo, 0.25, 0.5, and 1.0-mg). This double-blind study was designed to assess the effect of NTX on the analgesic effects of HYIR. The study evaluated the effect of 0.25, 0.5, and 1.0-mg of oral NTX on the analgesic effects of 15 mg HYIR in healthy volunteers with hyperalgesia. Three different treatment doses of NTX (0.25, 0.5, and 1.0-mg) were used in this study and compared with placebo NTX.

The same procedures were followed in each of the 4 treatment periods per treatment sequence. Subjects began each treatment period no less than 5 days after the end of the single-blind phase or a previous crossover period. The subject was trained on the use of the dolorimeter (used in Pain Latency testing) at the beginning of the first treatment period. Each subject entered the facility on the day of treatment after at least a 6-hour fast. Vital signs were taken and a urine drug screen, an alcohol screen, and a urine pregnancy screen (female subjects) were performed; all drug screening results had to be negative, for all drugs except the study medications, for the subject to continue.

A test site and a control site were selected on each forearm and marked with a washable marker. Next, the topical anesthetic was applied to the test site. After approximately 1.5 hours (allowing for the anesthetic to take effect), the cream was removed and a thermal stimulus was applied to the test site for 3 minutes using a copper mass heated to 52°C. After allowing approximately 1 hour sensory recovery, the following baseline measurements were conducted: vital signs, pupillometry, Thermal Discomfort (at 43°C, 46°C, and 49°C), Pain Latency (latency after application of a radiant heat stimulus), Symptom Rating Scale, Drug Rating Questionnaire, and Opioid-elicited Drug Effects Questionnaire.

Immediately after the baseline assessments were completed, each subject received 15 mg of oral HYIR and either placebo, 0.25, 0.5, 1.0-mg of NTX according to the randomization code. The same test measurements conducted at baseline were then conducted at 0.5, 1, 2, 3, and 4 hours postdosing. Thermal Discomfort and Pain Latency testing were conducted at each time point on the control site (area on the other forearm, which was not subjected to the thermal stimulus). Testing on the control site was done prior to the test site for each time point. Each subject rested and recovered for no less than 5 days before returning for the next period. In the follow-up phase, subjects returned to the clinic within 7 days after completion of the study for a final check of the test site and a review of the subject's laboratory data prior to official discharge from the study.

### Pharmacodynamic Measures

At each test session, the following pharmacodynamic parameters were recorded within 30 minutes before dose administration and at 0.5, 1, 2, 3, and 4 hours postdosing:
Thermal Discomfort testing (at 3 temperatures)
Pain Latency testing
Pupil diameter
Symptom Rating Scale Questionnaire
Opioid-elicited Drug Effects Questionnaire
Drug Rating Questionnaire

### Thermal Discomfort Testing Using Graded Thermal Stimuli

Thermal Discomfort testing was designed to measure a subject's perception of discomfort following a 5-second contact with a warmed 1" diameter copper mass (Uniformed Services University of the Health Sciences, Bethesda, MD), A thermal injury was induced by applying a heated (52°C) copper mass to the subject's forearm for 3 minutes. Thereafter, the test consisted of exposing the subjects to heated copper masses at 3 different temperatures: 43°C, 46°C, and 49°C. The required temperature of each copper mass was achieved by inserting the copper mass into a heating block (Models 145 and 147) (Fisher Scientific, Indiana, PA), which rested within an Isotemp Dry Bath (Fisher Scientific, Indiana, PA). The temperature sequence of the copper masses, applied at each measurement time point, was randomly selected for each subject, upon entry into the double-blind portion of the study. Thermal Discomfort testing was done both on control and experimental skin sites. Subjects rated their discomfort using a 100-mm VAS; the scale was anchored on the left with "No Discomfort at All" and on the right with "Most Intense Discomfort Possible for Me." Each subject responded by marking a vertical line on the horizontal scale between 0 and 100 mm. The distance from the left anchor to the vertical mark was measured and was used as the quantitative measure of Thermal Discomfort.

### Pain Latency Testing

The Pain Latency test was designed to capture the latency time, in seconds, from application of a radiant heat stimulus to the onset of pain as evidenced by self-termination of the radiant heat stimulus. The radiant heat stimulus was applied to both a control and an experimental skin site using a Model 33 Tail Flick Analgesia Meter (IITC Inc, Woodland Hills, CA). Each subject was trained to use this dolorimeter at the beginning of the first double-blind period. The dolorimeter was placed at a fixed distance of 4 inches from the subject's skin and emitted a high intensity light onto the selected skin site. The investigator turned the dolorimeter on; the subjects stopped the test (turned off the dolorimeter) by pressing the stop button at the onset of pain sensation. The total time that the subject was exposed to the high-intensity light was recorded onto the appropriate CRF page.

### Pupillometry

Pupillometry was performed to measure the effect of the study treatment on pupil diameter. The pupil was photographed using a Polaroid One-Step Closeup Camera (Polaroid Corporation, Cambridge, MA) with modified 2X magnification oculars (John Hopkins University, Baltimore, MD) and Polaroid 600 color film (Polaroid Corporation, Cambridge, MA). Background lighting in the examination room was measured using a Model L-246 sekonic LUX Meter (Sekonic Co, Tokyo, Japan). The camera was positioned on the subject's eye socket, aligning the iris with the middle of the opening of the lens adapter. The pupil diameter was measured from the photograph, in millimeters, using Model CD-6C Mitutoyo digital calipers (Judge Tool Sales, Southport, CT). The same eye was measured at all times for each subject.

### Symptom Rating Scale Questionnaire

The Symptom Rating Scale Questionnaire consisted of 25 items. For each item, the subject was instructed to indicate "How you feel right now." Each item was rated on a 3-point scale: "I don't feel this way at all," "I feel like this somewhat," or "I really feel this way." Twelve of the items were classified as agonist items and 13 were classified as antagonist items. Agonist items were symptoms associated with opioid administration. Antagonist items were symptoms associated with opioid withdrawal. The 12 agonist items were talkative, energetic, heavy/sluggish, carefree, itchy skin, happy, nervous, content, head nodding, relaxed, pleasant, and drifting. The 13 antagonist items were restless, sick to stomach, irritable, tense, jittery, hot or cold flashes, skin clammy or damp, face blushing, yawning, watery eyes, runny nose, chills/goose flesh, and sweating.

### Qpioid-elicited Drug Effects Questionnaire

Seven drug effects were rated using a 0 to 100-mm VAS scale anchored on the left by "None at all" and on the right by "An awful lot." These effects included nausea, vomiting, dizziness, drowsiness, constipation, itchiness, and dry mouth. The subject placed a vertical mark on the horizontal line at the distance that best corresponded to the way he or she felt from the drug at that moment.

### Drug Rating Questionnaire

Responses to 3 drug questions were rated using a 0 to 100-mm VAS scale anchored on the left by "Not at all" and on the right by "An awful lot." These questions were "Do you feel a drug effect now?", "Do you like the drug effect you are feeling now?", and "Do you dislike the drug effect you are feeling now?" The subject placed a vertical mark on the horizontal line at the distance that best corresponded to the way he or she felt from the drug at that moment.

### Adverse Events

An AE (adverse event) was defined as any unfavorable and unintended sign (including abnormal laboratory findings), symptom or disease temporally associated with the use of a medicinal product, whether or not considered related to the medicinal product. An AE was classified as a TEAE (treatment emergent adverse event) only if the AE occurred after the first dose of the study drug was administered to a subject who was enrolled in the study. The period of observation for TEAEs was from the time that the first dose of study medication was administered until release from the study after completion of period 4 or at early discontinuation. All AEs reported by the subject or observed by the investigator/study staff were fully documented throughout the study. When AEs were encountered that required medical intervention, appropriate supportive and/or definitive therapy was provided by appropriately qualified and licensed medical personnel. If any AE was not resolved at study completion/discontinuation, the subject was followed until resolution, until no further improvement was expected, in the opinion of the investigator, or until the subject could not be contacted.

### Pharmacodynamic Results

There were no statistically significant differences in mean Thermal Discomfort VAS scores, Pain Latency, or for the derived pharmacodynamic metrics of hyperalgesia between the HYIR + placebo NTX treatment and the HYIR + 0.25-, 0.5-, or 1.0-mg NTX treatments.

There were statistically significant increases in pupil diameter AUC (change from predose), but not PDmax, after administration of HYIR + 0.5mg NTX and HYIR + 1.0-mg NTX compared with HYIR+ placebo NTX.

In general, there were no statistically significant differences among the treatments or consistent NTX dose-related trends pertaining to the subjective opioid agonist effects of HYIR, as assessed by the Subjective Symptom Rating Scale and Subjective Drug Rating Questionnaire.

### Overall Conclusions

The following conclusions were drawn from the results of this study in healthy volunteers:

None of the doses ofNTX used in this study (0.25, 0.5, or 1.0-mg) produced a statistically significant reduction of the analgesia produced by 15 mg oral HYIR as measured by Thermal Discomfort VAS scores and Pain Latency testing.

There was no consistent NTX dose-related effect on the physiologic opioid agonist activity of the 15-mg dose of HYIR. However, all NTX doses reduced HYIR-induced pupil constriction.

There was no consistent NTX dose-related effect on the subjective opioid agonist activity of the 15-mg dose of HYIR.

There were no safety concerns associated with the treatment of healthy volunteers with 15 mg of oral HYIR combined with 0.25, 0.5, or 1.0-mg NTX.

The number of reported TEAEs, commonly associated with opioid use, decreased with increased NTX dose.

Many other variations of the present invention will be apparent to those skilled in the art and are meant to be within the scope of the claims appended hereto.

## Claims

1. A pharmaceutical composition comprising about 5 to about 20 mg hydrocodone or pharmaceutically acceptable salt thereof and 0.055 to 0.56 mg naltrexone or pharmaceutically acceptable salt thereof, said naltrexone or pharmaceutically acceptable salt thereof and said hydrocodone or pharmaceutically acceptable salt thereof in a ratio of from 0.011:1 to 0.028:1.

2. The pharmaceutical composition of claim 1 comprising about 5 mg hydrocodone or pharmaceutically acceptable salt thereof and from 0.055 mg to 0.14 mg of naltrexone or pharmaceutically acceptable salt thereof.

3. The pharmaceutical composition of claim 1 comprising about 7.5 mg hydrocodone or pharmaceutically acceptable salt thereof and from 0.0825 mg to 0.21 mg of naltrexone or pharmaceutically acceptable salt thereof.

4. The pharmaceutical composition of claim 1 comprising about 10 mg hydrocodone or pharmaceutically acceptable salt thereof and from 0.11 1 mg to 0.28 mg of naltrexone or pharmaceutically acceptable salt thereof.

5. The pharmaceutical composition of claim 1 comprising about 15 mg hydrocodone or pharmaceutically acceptable salt thereof and from 0.165 mg to 0.42 mg of naltrexone or pharmaceutically acceptable salt thereof.

6. The pharmaceutical composition of claim 1 comprising about 20 mg hydrocodone or pharmaceutically acceptable salt thereof and from 0.22 mg to 0.56 mg of naltrexone or pharmaceutically acceptable salt thereof.

7. The pharmaceutical composition of claim 1 comprising about 5 mg hydrocodone or pharmaceutically acceptable salt thereof and 0.0625 mg of naltrexone or pharmaceutically acceptable salt thereof.

8. The pharmaceutical composition of claim 1 comprising about 7.5 mg hydrocodone or pharmaceutically acceptable salt thereof and 0.09375 mg of naltrexone or pharmaceutically acceptable salt thereof.

9. The pharmaceutical composition of claim 1 comprising about 10 mg hydrocodone or pharmaceutically acceptable salt thereof and 0.125 mg of naltrexone or pharmaceutically acceptable salt thereof.

10. The pharmaceutical composition of claim 1 comprising about 15 mg hydrocodone or pharmaceutically acceptable salt thereof and 0.1875 mg of naltrexone or pharmaceutically acceptable salt thereof.

11. The pharmaceutical composition of claim 1 comprising about 20 mg hydrocodone or pharmaceutically acceptable salt thereof and 0.25 mg of naltrexone or pharmaceutically acceptable salt thereof.

12. The pharmaceutical composition of claim 1 further comprising a sustained release excipient which provides a sustained release of the hydrocodone or pharmaceutically acceptable salt thereof.

13. The pharmaceutical composition of claim 1 further comprising a sustained release excipient which provides a sustained release of the naltrexone or pharmaceutically acceptable salt thereof.

14. The pharmaceutical composition of claim 1 further comprising a sustained release excipient which provides a sustained release of the hydrocodone or pharmaceutically acceptable salt thereof and the naltrexone or pharmaceutically acceptable salt thereof.

15. The pharmaceutical composition of claim 12 and 14 wherein the dosage form provides effective pain relief for at least 12 hours after steady state oral administration to human patients.

16. The pharmaceutical composition of claim 12 and 14 wherein the dosage form provides effective pain relief for at least 24 hours after steady state oral administration to human patients.

17. The pharmaceutical composition of claim 14 wherein the hydrocodone or pharmaceutically acceptable salt thereof and the naltrexone or pharmaceutically acceptable salt thereof are substantially interdispersed in said sustained release excipient.

18. The pharmaceutical composition of claims 1-11 wherein said hydrocodone is in the form of the bitartrate salt.

19. The pharmaceutical composition of claims 1-11 wherein said naltrexone is in the form of the hydrochloride salt.

20. The pharmaceutical composition of claims 1-19 further comprising a non-steroidal anti-inflammatory drug selected from the group consisting of ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam, pharmaceutically acceptable salts thereof and mixtures thereof

21. A method of treating pain in a human patient comprising orally administering a pharmaceutical composition according to claims 1-20.

22. A method of preparing a pharmaceutical composition comprising combining about 5 to about 20 mg hydrocodone or pharmaceutically acceptable salt thereof and 0.055 to 0.56 mg naltrexone or pharmaceutically acceptable salt thereof inro an oral dosage form, said naltrexone or pharmaceutically acceptable salt thereof and said hydrocodone or pharmaceutically acceptable salt thereof in a ratio of from 0.011:1 to 0.028:1.

23. A method of deterring abuse of a hydrocodone formulation comprising preparing a pharmaceutical formulation of claims 1-20.

24. The use of hydrocodone or a pharmaceutically acceptable salt thereof, in the preparation of a dosage form according to any of claims 1-20.

25. The use of naltrexone or a pharmaceutically acceptable salt thereof, in the preparation of a dosage form according to any of claims 1-20.

26. The use of hydrocodone or a pharmaceutically acceptable salt thereof; and naltrexone or a pharmaceutically acceptable salt thereof, in the preparation of a dosage form according to any of claims 1-20.
